# EUROPEAN PATENT SPECIFICATION

(11) **EP 3 731 751 B1**
(45) Date of publication and mention of the grant of the patent: **19.06.2024**
(21) Application number: 18895132.1
(22) Date of filing: 28.12.2018
(51) Int. Cl.: G01N 33/543, G01N 33/74, G01N 33/68, C07K 16/26, G06T 7/90, A61B 5/145, A61B 5/00, A61B 10/00

(54) **SYSTEMS AND METHODS FOR TRACKING PROGESTERONE**
SYSTEME UND VERFAHREN ZUR VERFOLGUNG VON PROGESTERON
SYSTÈMES ET PROCÉDÉS DE SUIVI DE LA PROGESTÉRONE

(30) Priority: 28.12.2017 US 201762611467 P; 20.02.2018 US 201815900794
(43) Date of publication of application: 04.11.2020
(73) Proprietor: MFB Fertility, Inc., Boulder, CO 80301 (US)
(72) Inventor: BECKLEY, Amy, Erie, CO 80516 (US)
(74) Representative: J A Kemp LLP
(86) International application number: PCT/US2018/068027
(87) International publication number: WO 2019/133920

(56) References cited:
- WO-A1-94/04924
- WO-A1-98/39657
- WO-A1-2016/142610
- WO-A2-2007/049157
- WO-A2-2007/049157
- US-A- 4 450 239
- US-A1- 2006 121 626
- US-A1- 2006 121 626
- US-A1- 2015 094 227
- US-A1- 2015 094 227
- US-A1- 2018 321 251
- MFB FERTILITY, INC.: "How do Ovulation Double Check Tests work", YOUTUBE, 2 October 2017 (2017-10-02), page 1 pp., XP054979707, Retrieved from the Internet: URL:https://www.youtube.com/watch?v=HX-yNp DRGmg>
- MFB Fertility, Inc.: "How do Ovulation Double Check Tests work", , 2 October 2017 (2017-10-02), XP054979707, Retrieved from the Internet: URL:https://www.youtube.com/watch?v=HX-yNp DRGmg [retrieved on 2019-02-25]
- MESEN, TB et al.: "Progesterone and the Luteal Phase: A Requisite to Reproduction", Obstetrics and Gynecology Clinics of North America, vol. 42, no. 1, March 2015 (2015-03), pages 135-151, XP055709390,
- MUNRO, CJ et al.: "Relationship of Serum Estradiol and Progesterone Concentrations to the Excretion Profiles of Their Major Urinary Metabolites as Measured by Enzyme Immunoassay and Radioimmunoassay", Clinical Chemistry, vol. 37, no. 6, June 1991 (1991-06), pages 838-844, XP055624183,

## Description

### FIELD OF THE INVENTION

This invention relates to methods devices and test kits for use in confirming the occurrence of ovulation in female mammals, especially humans.

The invention is particularly, although not solely, concerned with simple practical procedures that can readily be applied by unskilled persons, e.g. in the home, to provide reliable information concerning confirmation of whether or not ovulation has occurred, whether progesterone levels are sufficient to support a pregnancy, and other information related to or deriving from the presence of progesterone in the body.

An embodiment of the invention may also be used by persons wishing to enhance the likelihood of conception by more clearly identifying the close of the fertile window, due to confirmation of ovulation. An embodiment may also be used by persons wishing to avoid miscarriage during pregnancy due to inadequate progesterone levels. In an embodiment, the invention is intended for use in the confirmation of ovulation, which is applied as an aid to the avoidance of unwanted pregnancy.

### BACKGROUND OF THE INVENTION

Analysis of the levels of hormone in an individual can provide important and helpful prognostic information. For example, detected hormone levels may enable an evaluation of a woman's health concerns such as fertility, pregnancy and menopause. One component in evaluating a woman's fertility status is assessing her ovulatory function. The menstrual cycle generally lasts 28 days. The first day of menses is traditionally identified as cycle day one. During the first half of the cycle, known as the follicular phase, the secretion of follicle stimulating hormone (FSH) by the anterior pituitary gland promotes follicle growth and secretion of both gonadotropins (FSH and luteinizing hormone, LH) stimulates estradiol (E2) synthesis and secretion by growing follicles. The exponential increase in E2 triggers the mid-cycle LH surge and ovulation, or release of the ovum from the dominant follicle. After ovulation, the remnants of the ruptured follicle become the corpus luteum (CL), a structure with a normal life span of 12 to 14 days, that produces E2 and progesterone (P4). P4 binds to endothelial cells within the uterus, preparing the uterus for embryo implantation. If fertilization does not occur, the CL degenerates as all trophic support is lost and circulating levels of E2 and P4 decline, causing the uterine lining to shed (menstrual bleeding). Therefore, there are very precise requirements for patterning of FSH, LH, E2, and P4 across the menstrual cycle to drive follicle growth, ovulation, and a normal luteal phase length.

Estrone-3-glucuronide (E3G) is a principal metabolite of E2. The urinary levels of E3G correspond to the serum levels of E2. Similarly, pregnanediol-3-glucuronide (PdG) is a principal metabolite of progesterone. The urinary levels of PdG are known correspond to the serum levels of progesterone.

If fertilization of the oocyte occurs, the implantation of the fertilized oocyte within the uterine endometrial lining triggers production of human chorionic gonadotropins (hCG) that maintains the corpus luteum and progesterone production.

A variety of lateral flow assay-based tests are available for in-home use to evaluate urine for the presence of hormonal analytes. Many known examples of tests configured to assess pregnancy evaluate urine to detect the presence of hCG, such presence indicating the onset of pregnancy. It is likewise known to use tests to estimate an ovulation date of a woman by testing urine for the presence of lutenizing hormone (LH). Exemplary tests are disclosed in US 9,206,254 B2. LH lateral flow assay-based tests have traditionally been used to estimate an ovulation date of a woman, wherein the urine comprises LH to be detected. These tests are configured such that a urine sample is applied to one end of the lateral flow assay. The urine, together with the labelled LH antibody, permeates through the strip material. The sample progresses into a detection zone in the lateral flow assay-based test. In the detection zone, a specific binding reagent for LH is mobilized. The LH, which is present in the urine sample, can therefore become bound within the detection zone. The amount of LH, which is bound in the detection zone, can then be optically observed by labelling reagents. Typical lateral flow assay-based tests also comprise a control line to convey an unrelated signal to the user merely confirming that the device has worked as intended.

A variety of lateral flow assay-based tests are previously known and available to consumers to help track some of the hormones involved in the female menstrual cycle. Such strips include lateral flow assay-based tests configured to detect FSH, LH and hCG. Other lateral flow assay-based tests include those configured to detect E3G and LH together and to interpret the results to give a digital reading to indicate low, high or peak fertility. However, there remains a need for a urine based at-home test strip 1001, optionally as a complement or improvement to the other at home urine based lateral flow assay-based tests associated with the female menstrual cycle, that can provide the consumer information regarding their progesterone levels by detecting and even quantitating PdG. Moreover, there remains a need to display the results of a specially configured progesterone test to be read, interpreted and displayed to a user, similar to how the results are displayed from lateral flow assay-based test configured to detect other hormones and their analytes. A major reason for the present lack of a progesterone test is the peculiar difficulty in detecting the presence of PdG in urine at threshold levels, in stark contrast to the relative general ease in detecting the presence of FSH, LH and hCG in urine. The difficulty in detecting the presence of PdG in urine derives from the unique chemical structure of PdG. Thus, lateral flow assay-based tests configured to evaluate for the presence of other hormones cannot be easily re-configured to evaluate for the presence of progesterone or its analytes.

Test strip 1001s for non-progesterone hormones can be evaluated with the assistance of a variety of complementary testing devices, such as those disclosed in United States Patent Applications US 2004/0253142 A1 and US 2013/0065321 A1, and those disclosed in United States Patent US 6,352,862 B1. In these devices, the user can directly view results derived from the detection zone and the control line of a lateral flow assay-based test configured to evaluate urine for the presence of non-progesterone hormones and their analytes. Such testing devices, however, do not have the capability to receive or evaluate results from lateral flow assay-based tests configured to detect for the presence of progesterone or progesterone analytes (such as PdG), which present in a significantly different manner. For instance, lateral flow assay-based tests configured to evaluate for the presence of LH or hCG present a colored line for a positive result. Previous attempts to present a colored line on a lateral flow assay-based test intended to display a line to indicate a positive result for the presence of PdG, in contrast, have proven unreliable and wildly inaccurate. Moreover, LH and hCG must be tested at different days in a menstrual cycle than the days for testing progesterone or progesterone analytes.

Further relating to lateral flow assay-based test configured to detect the presence of LH, FSH or hCG hormones, a method is known to illuminate the detection zone of a lateral flow assay-based test for LH, FSH or hCG hormones by utilizing light sources with monochromatic light or nearly monochromatic light to determine whether the intensity in the detection zone of such tests has reached a certain positive color threshold. In this case, a related device may be configured to output a respective positive or negative signal based on the presence of color on such tests in excess of a color threshold. Prior art devices teach methods for reading and interpreting tests for LH, FSH or hCG. Such methods include steps related to the estimation of an ovulation date via a smartphone device. Such a smartphone device is configured to obtain color data of successive tests and a processor, such processor configured to evaluate the color data obtained by the camera. However, such methods and smartphone devices, including the teachings described in European Patent Application EP 3,335,638 A1 filed Dec. 13, 2016, are not configured to or utilized to detect for the absence of color on a test, or color intensity below a certain threshold on a lateral flow assay-based test, such lacking color indicating a positive result for the presence of PdG.

In assessing ovulatory function, daily measurements of LH levels from test strip 1001s configured to detect the presence of LH may be used to predict the timing of ovulation. The use of urine tests to determine the presence of LH to predict ovulation is widely known. The rise in blood LH may occur roughly 24-36 hours prior to ovulation. The challenge, however, associated with reliance upon LH-based ovulatory prediction is the mere predictory nature of detecting the LH surge to only estimate future ovulation. In contrast, the only hormone that can be tested specifically to confirm that ovulation has actually already occurred is progesterone. In certain circumstances, for instance, a female's LH level may surge without subsequent ovulation. In contrast, the indication of progesterone to a certain threshold occurs only after ovulation. Thus, a test configured only to detect the presence of LH as widely known in the prior art does not sufficiently confirm ovulation.

Previously known tests have not been successfully configured to reproducibly and consistently interpret results for the presence of progesterone or its analytes in urine. Previous attempts to standardize the configuration of tests and methods for evaluation of urine for progesterone or its analytes heretofore have led to unreliable and difficult-to-interpret results. An adequately formulated configuration for a reproducible and consistently accurate urine-based test for progesterone or progesterone analytes remains to be discovered. Previous attempts to configure a test to detect progesterone and/or its analytes have proven ineffective and unreliable.

Relatedly, it would be desirable and an unmet need to have an effective device for confirming, rather than merely predicting the onset of ovulation (as is available via tests configured to evaluate urine for the presence of LH), via a test configured to confirm for the presence of progesterone and/or its analytes in urine. It remains desirable and an unmet need to have an effective test as an improvement to existing complementary test readers, which are configured to evaluate for the presence of non-progesterone hormones in urine, configured to allow existing complementary test readers to evaluate for the presence of progesterone or its analytes in urine. As past ovulation would indicate the onset of a woman's infertile phase, there remains a need for utilization of such an improvement in association with previously unrealized benefits in associated with birth control. Further, it would be desirable to utilize a respective method, leading to more accurate results than those displayed the previously known devices, and which are able to confirm (rather than merely predicting) ovulation via a clear digital reading displayed upon an associated complementary reader, optionally comprising a smartphone device.

Currently, a commonly utilized method to self-confirm ovulation without hormone evaluation is through the tracking of basal body temperature (BBT). In association with such method, a female's temperature is tracked shortly after awaking in the morning on a daily basis. When the temperature significantly rises, by 0.5-1 degree Fahrenheit or more, ovulation is indicated. A variety of challenges exist with such method, including that body temperature fluctuations could be caused by sources unrelated to ovulation, thereby rendering the results uncertain. Moreover, the BBT method is widely regarded as time consuming, difficult to measure, and often inaccurate. Thus, it remains desirable to have a more accurate mechanism than the BBT method to detect for the presence of progesterone or its analytes, at least as convenient to women as the BBT method, to confirm ovulation.

A wide variety of alternative techniques have been demonstrated in the art to detect ovulation, some relying on the monitoring of one or more parameters which alter as the event of ovulation approaches. Typical parameters which have been invoked are the detection of concentration of a body fluid analyte, such as estradiol and metabolites thereof (i.e. estrone-3-glucuronide (E3G)) beyond a certain threshold. Other associated methods that have been used are testing for a surge in LH, tracking BBT and tracking various physiological changes such as the characteristics of vaginal mucus. A challenge associated with such techniques is that they can only provide predictive and/or imprecise information, including information merely associated with anticipated future events in the menstrual cycle. LH tests do not allow a woman to reliably assess whether she is in an infertile period or otherwise in a risk period for pregnancy in case of unprotected intercourse. By contrast, only the presence of progesterone in a woman's body may actually confirm ovulation, which indicates the start of the infertile period. Resultantly, such non-progesterone tests are of limited and uncertain use, particularly in association with the avoidance of unwanted pregnancy as further discussed below.

Moreover, to provide reliable information concerning fertility status for purposes associated with the avoidance of pregnancy, the user must be given confirmation with a high degree of confidence of the onset of the infertile phase in the cycle. When a woman produces progesterone of a threshold amount, ovulation is confirmed. In a female, the production of progesterone is well understood to coincide with ovulation. Therefore, ovulation is confirmed upon receiving a positive progesterone test, which in the prior art has generally only been available via a blood (serum) test. Once ovulation has been confirmed via a positive serum progesterone test, a female's fertile window has closed. Many methods for the detection of ovulation are well known, including those described in "Detection of ovulation, a review of currently available methods" Bioeng Transl Med. 2017 Sep; 2(3): 238-246. Available at: https://www.ncbi.nlm.nih.gov/pmc/articles/PMC5689497/). As a result, those seeking to engage in unprotected sexual intercourse after ovulation may generally do so for the remainder of that menstrual cycle without the possibility of pregnancy because the woman is in her non-fertile stage.

Previously known progesterone tests configured to evaluate blood serum often require a physician order and a laboratory evaluation of blood. A related problem is that such a process is a time consuming and costly mechanism for testing the presence of progesterone. As a result, progesterone tests accomplished via the evaluation of blood serum are generally impractical for many uses deriving from progesterone testing, including such uses as the avoidance of pregnancy. Such cumbersome processes also complicate determination of whether low progesterone exists during pregnancy threatening a miscarriage. Therefore, an improved, more convenient, and less costly mechanism for the evaluation for the presence of progesterone in a woman's body is highly desirable and remains an unmet need.

Measurements of serum progesterone levels 18 to 24 days after the onset of menses or seven days before the next cycle may be used to confirm whether ovulation had occurred. In the event of a confirmed pregnancy, serum progesterone levels may be utilized to assess nonviable pregnancies, e.g., ectopic pregnancy or spontaneous abortion (miscarriage). In pregnant individuals, a serum progesterone value of 25ng/ml is 98% of the time associated with a viable pregnancy, while a value of < 5 ng/ml identifies a nonviable pregnancy. Physicians typically consider 10-12 ng/ml to be minimal amount needed to sustain a pregnancy. However, the procedures associated with drawing a female's blood to determine progesterone levels in association with the determination of ovulation, fertility planning, the assessment of the viability of pregnancy, to assess the onset of menopause and to engage in activities related to pregnancy avoidance render testing for serum progesterone impractical. For instance, timing ovulation via serum progesterone testing for fertility planning typically requires the passage of several days prior to receiving test results. Therefore, an improved test configured to evaluate progesterone levels capable of being performed without laboratory work, and capable of providing faster time to results, is highly desirable and remains an unmet need.

In accordance with purposes associated with the avoidance of pregnancy, access to information confirming that ovulation either has or has not occurred heretofore has been unavailable via an improved and effective urine based progesterone test. Thus, an effective and accurate test to evaluate urine for the presence of progesterone or its analytes would be highly desirable to assist the woman in association with pregnancy avoidance purposes. This is especially true as many well-known means for pregnancy avoidance and birth control are associated with the ingestion of hormones that have been linked with health problems, particularly after long term usage. Thus, there remains a need for chemical and hormone free means of birth control and pregnancy avoidance, such as an easy and accurate way to confirm that ovulation has already occurred.

Diagnostic tests for screening analytes, e.g. urinary hormones or metabolites thereof, may utilize antibodies specific to the analyte. A change in the level from a predetermined threshold level may be noted by differences in color or color intensity compared with the color in a reference window or a reference guide. The color change may be produced using techniques such as enzyme-linked immunosorbent assays or lateral flow color matching assays to indicate the amount of analyte in a urine sample. It is known that the presence of PdG in urine corresponds to the presence of progesterone in blood.

Moreover, others have unsuccessfully attempted to create a urine-based test to provide information regarding progesterone levels by detecting and quantitating PdG in a context other than in a lab environment, such as for at home for use by a non-expert user. Such attempts have proven fruitless due to inaccuracies associated with the tests with regard to the precision of detection and measurement of PdG. Further, the tests based on the detection of PdG in urine are complex, difficult to implement. Some require the preliminary study of at least one menstrual cycle prior to reliance on the test. Further, the optimal chemistry to enable the creation of simple, user-friendly test to evaluate urine for PdG remains to be discovered. Specifically, it has yet to have been discovered how to create a progesterone test result discernable by the naked eye, despite years of effort. Currently, progesterone tests are generally limited to use within a lab environment. Colorimetric lab-grade electronic readers are used to detect differences in color otherwise imperceptible to the naked eye. Such lab-based tests determine concentrations to a high accuracy, often with the assistance of lasers. There remains an unmet need for a test for progesterone or progesterone analytes configured to allow a person to visually review the results with the naked eye or by readers affordable to consumers and without the assistance of lab-grade equipment, an alternative solution.

Previous attempts to create a lateral flow assay for detecting progesterone metabolites in urine, including the inventive matter disclosed in United States Patent 6,924,153 granted on August 2, 2005, the inventive matter disclosed in United Kingdom Patent Application Publication No. GB 2,204,398 A as published on November 9, 1988, and similar prior art items, were unsuccessful due to the technical difficulties and inappropriate selection of component antibodies (namely the selection of component antibodies of improper isotypes) and the complexity associated with the assay system. It remains an unmet need, therefore, to create a lateral flow assay configured with antibodies of the proper isotypes to effectively detect progesterone metabolites in urine.

In certain cases, such technical difficulties also were associated with the development of antigen and antibody chemistries of such ratios, component parts and/or elements to specifically produce visual results readable to the naked eye. Other prior art matter, for instance the subject matter disclosed in PCT Patent Application WO 2016/142610 to Van Der Geest and Ulmann, published on September 15, 2016, discloses Bovine Serum Albumin (BSA) as the carrier protein utilized in association with a test configured to detect PdG. However, the utilization of BSA as a carrier protein is inadequate for usage in a urine-based progesterone or PdG testing solution intended to display results visible and discernable to the naked eye. This is because the utilization of BSA as the carrier protein results in a testing solution lacking the ability to adequately bind to colloidal gold (or other visual label), thereby resulting in a test delivering results that are problematically imperceptible to the naked eye. The resultant test incorporating BSA is ineffective and highly unreliable. It is therefore an unmet need to have a test to evaluate for the presence of progesterone configured by utilization of a carrier protein other than BSA that has the capability to bind to a visual label, said configuration allowing for a naked eye to read the results to the necessary usable perception level.

The specific type of carrier protein and specific antibody isotype chosen are known to be critically important in association with the utilization of assays for the detection of analytes in urine. The specific carrier protein and specific antibody isotype associated with the Van Der Geest and Ulmann disclosure do not result in an effective combination to evaluate urine for the presence of PdG. These and other prior art attempts have failed to result in a device that reliably and reproducibly produces enough color intensity to deliver clear and easily interpreted test results to confirm the presence of PdG for users with minimal training and a lack of specialized equipment. Therefore, an unmet need remains for a lateral flow assay configured to detect progesterone metabolites in urine by reliably and reproducibly delivering enough color intensity to portray clear and easily interpreted test results for users with minimal training and a lack of specialized equipment. A related unmet need remains to create an improved test strip configured to incorporate one or more carrier proteins other than BSA able to more effectively bind to a visual label. Moreover, there is a further unmet need to a test strip configured to incorporate antibodies of a specific isotype conjugated such that they have the capability to determine the presence of progesterone in a female via the presence of PdG in her urine and perceptibly present the results to a layperson user.

Another attempt to create a reproducible test to evaluate urine for the presence of progesterone analytes was disclosed in United States Patent US 6924153 B1, issued to Quidel on August 2, 2005. The test disclosed in the Quidel reference consists of a lateral flow assay with a three zone test system. The first of the three zones consists of the receiving zone, in which a urine sample potentially containing PdG and a labeled reagent is placed. The second of the three zones, is the barrier zone consisting of an invisible line that functions as a reagent sink. The reagent sink disclosed within Quidel is configured to absorb a pre-set amount of PdG of predetermined concentrations. The reagent sink line is not viewable on the test device described in Quidel, and thus not read for a positive or a negative reading. As the fluid applied to the lateral flow assay in the Quidel reference travels past the reageant sink, PdG is absorbed in an amount not exceeding the pre-determined threshold concentration of PdG. The Quidel reference specifically describes pre-set concentrations ranging from 2.5 µg to 100 µg. As the fluid then progresses over the second of the three zones, if any PdG remains that has not been absorbed into the sink line, it interacts with the third of the three zones, consisting of the capture zone, and then produces a positive test.

Many previously unaddressed problems are associated with the configurations taught in the Quidel reference. First, the complexity of the three zone test disclosed in Quidel renders it difficult to reproduce. Moreover, the difficulty in reproduction has been observed to have been caused in part by the difficulty in controlling the precise amount of PdG bound into the sink. Previously obtained inaccurate and inconsistent results associated with the Quidel test may have occurred because of its utilization of a barrier zone or reagent sink. Therefore, an improvement upon the urine PdG test described in the Quidel reference, featuring fewer binding zones, and that has a reduced risk of interference within an assay, remains an unmet need.

Researchers utilized the test disclosed in Quidel and published observed results in a publication entitled "Use of Urinary Pregnanediol 3-gluconoride to Confirm Ovulation" by Ecochard et. al., Steroids 2013 Oct., 78(10): 1035-1040. In this publication, they described inconsistent and inaccurate results in association with the use of the test described in the Quidel reference during the evaluation of urine for PdG. In that study, the researchers utilized a three zone test to attempt to visually quantify a PdG amount in a urine sample applied to the test disclosed in the Quidel disclosure. Further in the study, the authors suggested that only upon three consecutive positive results for PdG would ovulation be indicated, due to the high incidence of false PdG positive reads associated with the test disclosed in Quidel (variously referred to herein as the "Quidel test"). Specifically, the paper stated that "the [Quidel] PdG test had limited specificity to PdG because it would occasionally read positive before ovulation would take place." Due to its inaccuracy and inconsistency, the Quidel test is moreover therefore not a useful tool for tracking ovulation. Therefore, a need remains to create a urine PdG test that allows for a simpler configuration and that allows for precision with the amount of PdG that binds to the testing zone. Moreover, a need remains for an improved test to evaluate urine for the presence of PdG of a sufficient level of specificity to avoid false positive results associated with previous attempts, including the tests disclosed in United States Patent US 6924153 B1 which is further described above.

Prior art solutions are associated with challenges stemming from problematic antibody and PdG carrier protein (also referred to as the critical reagents or hormone binding partners) conjugation, selection and incorporation, often due to the conjugation, selection and incorporation of improperly chosen antibodies, antibody isotypes, conjugation techniques, or carrier proteins. A problem associated with prior art solutions is that the specifically chosen critical reagents with such solutions are undesirably cross-reactive. In certain cases, wrongly chosen antibodies have suboptimal affinities for the application of a PdG test. The wrongly chosen antibodies in prior art solutions are outside of a desired detection range. For instance, the wrongly chosen antibodies may result in a test that is not sensitive enough to allow a user to distinguish a positive and negative result. Sensitivity in such context may derive from suboptimal levels of affinity, avidity and specificity. In prior art tests where suboptimal sensitivity results from suboptimal specificity, the chosen antibody having a particular antibody isotype binds on items other than a PdG target. A problem with prior art tests having a particular suboptimal combination antibody, antibody isotype and/or carrier protein, is that the antibody and the PdG conjugate do not bind with the precision necessary to produce a viable, reproducible test result useful to detect the presence of PdG. An unmet need remains, therefore, for a test strip, comprising a more optimal combination of antibody, antibody isotype and/or a carrier protein, able to produce a viable, reproducible test result to detect the presence of PdG.

Improvements to previously known diagnostic tests for monitoring hormones in urine have been made by removing the step which required the individual to interpret the results. However, the previously known diagnostic tests have not been improved to evaluate urine for progesterone or its analytes. In these prior art products, electronic sensors and displays provide clear outputs indicating analyte levels. For example, the Clearblue^{®} Easy Fertility Monitor (CBEFM) provides a method for monitoring the fertility status of an individual using two hormones: LH and E3G with electronic reading and a digital display. The Clearblue^{®} Digital Ovulation Test (CDOT) is another commercially available device, which employs a variable threshold for LH surge determination, and is also an improvement over the color matching visually read tests. Similarly, the disclosures of United States Patent 6,451,619, which is properly characterized as a "test kit" in association with the below inventive disclosures, utilizes a reader/monitor to analyze analytes in urine. In addition, the disclosures of United States Patent Application 2012/0321519 A1, which is properly characterized as a "test kit" in association with the inventive disclosures below, relate to a fertility monitoring system. Moreover, the First Response^{®} Advanced Digital Ovulation Test similarly provides information related to monitoring LH with an electronic display. Moreover, the disclosures in United States Patent 9,063,091 to Tsai and Chen, which are properly characterized as a "test kit" in association with the inventive disclosures below, utilizes a computing device incorporating a camera in association with a test strip to evaluate for the presence of analytes. Moreover, the disclosures in United States Patent 9,939,385 to Nazareth et. al, which are properly characterized as a "test kit" in association with the inventive disclosures below, discloses apparatuses and a method to detect analyte levels from fluid samples. Although such disclosures, including the CBEFM, CDOT and First Response tests show improvement over the previously presented art, there still remains a need for a diagnostic device that is affordable to the average consumer and provides increased simplicity of use in comparison to devices known the prior art.

Moreover, as none of the tests display information related to progesterone or its analytes, a need remains for such diagnostic devices that are affordable to the consumer to simply display whether ovulation has occurred via analysis of urine for progesterone or its analytes. Further, none of the tests provide information indicating whether if ovulation has occurred, one can engage in unprotected sexual intercourse without risking unwanted pregnancy, whether progesterone levels are sufficient to support a pregnancy, or whether menopause has occurred. Previously known implementations of progesterone and PdG urine testing strips have been of insufficient quality to effectively work in conjunction with the electronic sensors and displays described above. Accordingly, improvements in detection systems are desirable, particularly in relation to the detection of PdG in urine, and to the interpretation and display of such results. Relatedly, there remains an unmet need for a device capable of evaluating urine for the presence of PdG and utilizing the results to provide an indication to a user whether she can engage in unprotected sex without risking unwanted pregnancy.

Understandably, because the severe consequence of imperfect advice concerning fertility status may be an unwanted pregnancy, the tendency has been to exercise extreme caution and to require testing of the relevant parameter or parameters throughout the cycle, and particularly right from the onset of the cycle (onset of menses). From the individual user's point of view, it would be advantageous if the necessity for such constant testing could be avoided and, instead, for the testing to be performed over a comparatively brief portion of each cycle. Not merely may this benefit the user in terms of convenience, but the cost of the method may also be reduced if the method utilizes disposable testing devices and only a few such disposable testing devices are required each month. Therefore, there remains and unmet need for disposable testing devices configured to evaluate urine for the presence of PdG and display the results.

### OBJECTIVES OF THE INVENTION

An objective of the present invention is to provide the option of basing an effective monitoring system solely, or at least primarily, on the presence of pregnanediol (PdG) in urine. Other advantages of the invention will be apparent from the following description.

An objective of the invention is to present a reproducibly functioning version of a specially configured test strip 1001 to evaluate urine for the presence of one or more metabolites of progesterone, such as PdG. Embodiments of the invention are configured for use in association with a reader device. An embodiment of the invention constitutes an improvement to an existing test strip 1001 reader device. The invention is characterized by the incorporation of the conjugation of a globulin of a specific type with a mouse anti-PdG antibody selected from a specific group of isotypes.

An objective of the invention is to provide a progesterone metabolite testing system characterized by the minimization of the burden on the user to interpret results, while still providing the user with worthwhile information, including information indicating that ovulation has actually occurred. The present inventor has recognized that prior art systems and mechanisms merely predict the occurrence of ovulation by testing for the presence of hormones other than progesterone or its analytes. Thus, embodiments of the invention are characterized by a system configured to evaluate for the presence of PdG in urine with an improved, effective test strip 1001. Embodiments of the invention function to minimize a user's testing burden, enabling the user to receive information useful to pregnancy avoidance, the closure of the most fertile period, and other information deriving from the confirmation of ovulation without requiring laboratory results.

Another objective of the present invention is to provide a method and devices for determining the presence and/or concentration of PdG and one or more other analytes in a single urine sample. Such method and devices allow for information related to the ovulation predictor hormones and their analytes, including LH and E3G, and information related to the ovulation confirming hormone, progesterone, and analytes of progesterone including PdG, to be collected in the same urine test. In an embodiment of the invention, labels (such as colloidal gold) are varied, with a separate and distinct label configured to attach to a separate hormone. In such embodiment, the present inventor has recognized the advantage that the test strip 1001 is configured to provide a different color for each hormone analyte indicating either the presence or absence of each hormone analyte following application of urine to the test strip 1001. In an embodiment, the test strip 1001 is configured to comprise a conjugate pad (also referred to as the "receiving zone") comprising anti-PdG antibody-collodial gold conjugate, and at least one other conjugate. In an embodiment, the at least one other conjugate comprises anti-LH antibody-conjugated with a different label, optionally differently colored latex beads.

The present inventor has recognized that LH and HCG commonly exhibit cross-reactivity, specifically due to the fact that HCG can bind to LH antibodies. Therefore, having different colors corresponding to the presence of different hormones provides a benefit by allowing an observer to determine whether cross-reactivity has taken place. In other words, if an area designated to test for the presence of LH displayed the coloration of the label for HCG, one skilled in the art would understand such a read to indicate that cross-reactivity has been demonstrated and that the test therefore is invalid. Alternatively, the present inventor has noted that due to the similarities in structure between estrogen analytes and progesterone analytes (in at least one example, said progesterone analytes consisting of PdG), cross reactivity could take place between those two hormone metabolites specifically. Thus, it is beneficial to have different hormones or hormone metabolites labelled with different colors. Such labelling taking place in an embodiment by binding to colloidal gold and/or one or more differently colored latex beads, and is therefore a teaching of an embodiment of the invention.

It is a further objective of the invention to provide such a multi-analyte assay method/device in which a particulate direct label is used to reveal the result of all assays, where one of the labels reveals the presence or lack of PdG, in association with the testing area specifically configured in accord with the teachings disclosed herein. The use of particulate direct labels is already known in simpler assay systems. Sometimes the use of particulate direct labels enables the assay result to be evaluated easily by eye. This may also be the case in assays in accordance with the present invention, although it is envisaged that in general the results of the assays will more conveniently and effectively be evaluated instrumentally.

A yet further object of the invention is to provide an assay method/device in which multiple analytes, wherein one of the analytes is PdG, in a single sample liquid can be determined accurately in a strip-format assay device which is interpreted instrumentally using electromagnetic radiation (eg. light) passed through the thickness of the assay strip. The strip material can be translucent or transparent. The extent of binding of particle labels in a detection zone in the strip can provide a quantitative assay result, because the particles can block light or other radiation and therefore reduce the transmission of the radiation through the strip, which in the case of a PdG evaluation indicates a negative test result.

Another objective of the invention is to produce improved combinations of assay result reading devices and associated sample testing devices which can provide accurate quantitative assay information, at least related to the presence of PdG, in a simple, quick and cost-effective manner.

Another objective of the invention is to display unified results of the urine evaluation for a variety of hormone analytes in addition to PdG, featuring the specially configured analyte testing area with results of LH, FSH and/or E3G testing in addition to PdG testing on a single test strip 1001.

A further objective of the invention is to display one or more testing results obtained via the application of urine to a test strip 1001 on an associated electronic device featuring a display, such associated electronic device optionally comprising a smartphone or dedicated electronic unit, indicating to a user at least whether or not a woman has ovulated.

An objective of the present invention is to provide information to a woman to indicate whether the woman has entered her infertile period and therefore may engage in sexual intercourse with a minimized risk of unintended pregnancy, and thereby engage in birth control without the need to ingest hormones.

### SUMMARY

The systems, methods, and devices of the disclosure each have several innovative aspects, no single one of which is solely responsible for the desirable attributes disclosed herein.

A method of detecting pregnanediol (PdG) of a predetermined level in a urine sample via a device comprising a specially configured test strip 1001 from an individual is provided. The test strip 1001 in embodiments of the invention derives from lateral flow technology and techniques in which urine samples are placed on one end of the strip and flow laterally towards the other end through a series of zones to product an end result. Lateral flow tests, also known as lateral flow immunochromatographic assays, are simple paper-based devices intended to detect the presence of a target analyte in liquid sample without the need for specialized and costly equipment. As used herein, the term "test strip" refers to a specially configured lateral flow assay or lateral flow immunochromatographic assay. A further method of detecting a variation in a monitored PdG level in a urine sample via a device comprising a specially configured test strip 1001 from an individual is also provided. Such further method includes collecting a series of samples over a single biological cycle of the individual, determining a baseline from a plurality of samples in the series, determining a threshold associated with the PdG level for the individual based at least in part on the determined baseline, generating a signal representative of the PdG level in one or more samples in the series, comparing the signal to the threshold, and generating an output based at least in part on a result of the comparing. In an embodiment, other hormones such as LH and E3G may be measured simultaneously with PdG via a system comprising a specially configured single test strip 1001 capable of evaluating urine for the presence of PdG and other hormones and analytes, especially LH and E3G.

A test kit for detecting PdG of a predetermined level, or otherwise detecting variation in a PdG level in a urine sample from an individual is provided. The test kit includes urine sample collectors and a reader. The reader includes a port for accepting a urine sample collector therein and a circuit. The circuit may be configured to determine a baseline from a plurality of initial samples in a series of samples collected with a series of urine sample collectors, determine a threshold associated with the PdG level for the individual based at least in part on the determined baseline, generate a signal representative of the PdG level in one or more samples in the series collected subsequent to the initial samples, compare the signal to the threshold, and generate an output signal based at least in part on the result of the comparing. In an embodiment, the test kit comprises a specially configured single test strip 1001 capable of evaluating urine for the presence of PdG and other hormones and analytes, especially LH and E3G.

Details of one or more implementations of the subject matter described in this specification are set forth in the accompanying drawings and the description below. Other features, aspects, and advantages will become apparent from the description, drawings, and claims. Note that the relative dimensions of the following figures may not be drawn to scale.

The invention is defined by the appended claims.

### BRIEF DESCRIPTION OF FIGURES

Fig. 1 depicts a test strip 1001 comprising a control line and one testing zone configured to detect the presence of PdG beyond a specific threshold in an embodiment of the invention, indicating a negative result as displayed.
Fig. 2 depicts a test strip 1001 comprising more than one testing zone, configured to detect for the presence of PdG beyond a specific threshold and two additional testing zones configured to detect for the presence of other analytes and/or hormones each beyond a specific threshold, in addition to a control line in an embodiment of the invention.in an embodiment of the invention.
Fig. 3 depicts a testing zone 1002and a test stick, the test stick positioned prior to installation into the stand-alone digital reader.
Fig. 4 a testing zone 1002and a test stick, the test stick installed into the testing zone 1002
Fig. 5 depicts a testing zone 1002and a smartphone during utilization to wirelessly send results of the test strip from the testing zone 1002to the smartphone.
Fig. 6 depicts a linear relationship of PdG concentration in urine versus color displayed on a test strip 1001.
Fig. 7 depicts a non-linear relationship of PdG concentration versus color displayed on a test strip 1001.
Fig. 8 depicts a digital reader configured to image a test strip comprising a control line and one testing zone configured to detect the presence of PdG beyond a specific threshold in an embodiment of the invention.
Fig. 9 depicts a digital reader configured to interpret a test strip comprising a control line and one testing zone configured to detect for the presence of PdG beyond a specific threshold and two additional testing zones configured to detect for the presence of other analytes and/or hormones each beyond a specific threshold an embodiment of the invention.
Fig. 10 depicts a method of use of a test strip zone configured to detect for the presence of at least one metabolite of progesterone in urine in an embodiment.
Fig. 11 depicts a method of use of a test strip configured to detect for the presence of at least one metabolite of progesterone in urine to avoid pregnancy while still engaging in sexual intercourse in an embodiment.
Fig. 12 depicts a method of use of a test strip configured to detect for the presence of at least one metabolite of progesterone in urine to confirm the onset of menopause in an embodiment.
Fig. 13 depicts a method of use of a test strip configured to detect for the presence of at least one metabolite of progesterone in urine to determine sufficiency of progesterone during pregnancy and to mitigate lack of sufficient levels of pregnancy in an embodiment.
Fig. 14 depicts a method of use of a test strip configured to detect for the presence of at least one metabolite of progesterone in urine to determine the status of progesterone supplementation in an embodiment.

### DETAILED DESCRIPTION

An embodiment of the invention consists of a test strip 1001 configured to evaluate urine for the presence of pregnanediol (PdG) in a concentration above a pre-defined threshold.

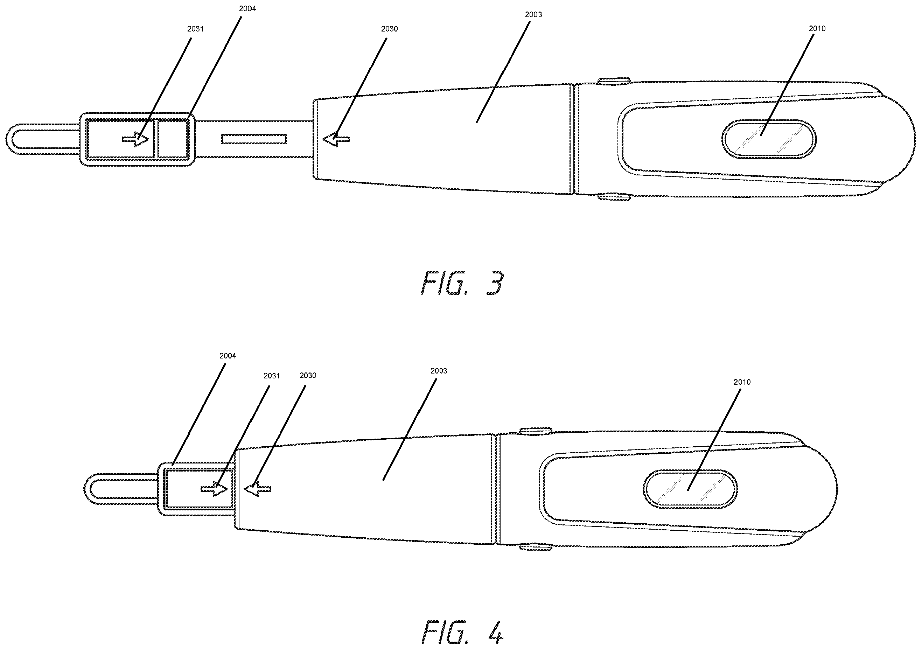

Aspects of the preferred embodiment of the invention are directed to a pregnanediol (PdG) urine lateral flow assay, also referred to herein as a "test strip." Embodiments of the test strip 1001 are characterized by having specific reagent combinations as described further herein, said specific reagent combinations uniquely enabling a strong enough interaction in the testing zone 1002 (also variably referred to as the "capture zone") of the test strip 1001 to allow for visual, naked eye inspection of the test results. Embodiments of the test strip 1001 are characterized by having specific reagent combinations as described further herein alternatively described as strong enough interaction in the testing zone 1002 of the test strip 1001 of the test strip 1001 for detection by a reader, said reader optionally forming part of a test kit. In an embodiment, the test kit further comprises a digital reader, optionally consisting of a stand-alone digital reader 2003 as depicted in Figs. 3-4.

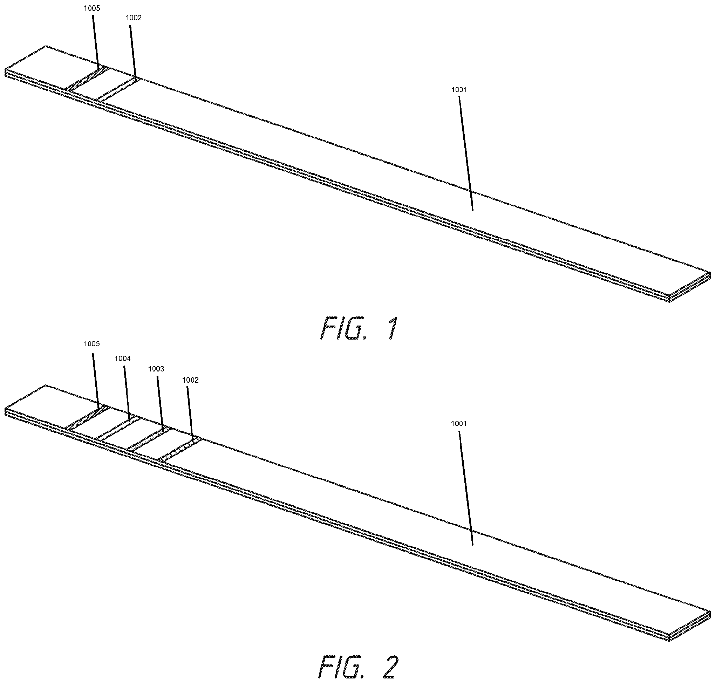

In an embodiment, the test strip 1001 includes a nitrocellulose portion. As the urine sample placed at the distal end of the test strip 1001 opposite the control line 1005 continues along the test strip 1001, the sample next encounters a testing zone 1002. In the example shown in Figure 1, the testing zone 1002 consists of an area for binding antibodies specifically necessary for detecting PdG as described further below. In the preferred embodiment, the testing zone 1002 is designed to trap unbounded antibodies that have not attached to PdG within the urine sample. In the preferred embodiment, the testing zone 1002 will thus become lighter as less of the complexes are accumulated. In an example implementation where the conjugate comprises colloidal gold, the color of the colloidal gold becomes apparent in the context where complexes of PdG are accumulated. In an alternative embodiment, an external reader comprising an electronics system, which may include sensors and/or a processor for performing a transformative algorithm on sensed data, may measure the colloidal gold specifically bound at the testing zone 1002 of the test strip 1001. Within an embodiment of the invention presented herein, the present inventor has created a novel composition of specific antibodies conjugated to a visual label, and pregnanediol conjugated to a carrier protein, at a specified set of ratios, to effectively display a consistent result during utilization of the test strip 1001, which is further described herein.

After the testing zone 1002, the test strip 1001 may include a control line 1005. The control line 1005 may also generally be referred to as a reference line. When present, the control line 1005 includes anti-mouse antibodies or other proteins that specifically bind the gold conjugated antibody. In embodiments, such configuration may be interpreted to provide a measurement of gold-bound antibody in the fluid that is not specifically bound to the analyte being tested.

In embodiments of the invention, a digital reader 2001 is configured to measure reflectance measurements from the testing zone 1002. In embodiments, the control line (also variably referred to as the "reference line") 1005 may be used separately as a reference to assist the digital reader 2001 to define successful testing and PdG concentrations. In alternative embodiments, the test strip 1001 is configured without a control line, as the digital reader 2001 would have an alternative mechanism to ensure the assay completed correctly. In an embodiment, such alternative mechanism consists of the optical determination by the digital reader 2001 that fluid has passed a predetermined point on the assay indicating completion. In some embodiments, the reflectance of light from the testing zone 1002 may be compared by the digital reader 2001 with the reflectance from the test strip 1001 downstream from the testing zone 1002 in a region where there may or may not be a control line 1005 to define successful testing and PdG concentrations. The present inventor has recognized that a test strip 1001 without a control line or reference line may be advantageous because it eliminates the need for the antibodies at this line, reducing cost of the test strip 1001.

In the first part of Fig. 1, a test strip 1001 is shown onto which a urine sample is applied. A first end of the test strip 1001 is soaked with urine in a cup around 5 seconds. After that, the test strip 1001 is to be left in the horizontal position to develop a result for up to 15 minutes. Subsequently, the test strip 1001 is read by either the naked eye, or a digital reader 2001, for estimation of the progesterone and/or PdG level for that given day. The test strip 1001 comprises at least one testing zone 1002, which is configured to provide an indication of progesterone and/or PdG presence and concentration in the urine sample. In an embodiment, the test strip 1001 also comprises a control line 1005, into which the applied sample of urine permeates to indicate that the test strip 1001 has been successfully prepared. In an embodiment, the test strip 1001 also comprises a blank zone onto which no testing result is provided. In various embodiments, the digital reader 2001 may interpret the result of the testing zone 1002 as a measure of the variation of the color contained within the testing zone 1002 as compared to the blank zone.

In an embodiment, the test strip 1001 comprises a nitrocellulose portion, which may terminate with a second overlapping region. The second overlapping region may serve as a border between the nitrocellulose portion and an absorbent portion of the test strip 1001. The absorbent portion of the test strip 1001 facilitates the uptake of the fluid sample as it arrives at the end of the test strip 1001.

The configuration of the test strip 1001 in its preferred embodiment comprises at least one testing zone 1002. In an embodiment, the at least one testing zone 1002 is created by striping a PdG-carrier protein conjugate onto the membrane of the test strip 1001. In its preferred embodiment, the test strip 1001 is configured to determine the presence of PdG in urine at a specific pre-defined threshold. The specific pre-defined threshold is implemented to the test strip 1001 by the application, in the proper ratio, of the amount of PdG-carrier protein conjugate to the testing zone 1002 of the test strip 1001, and the anti-PdG-collodial gold conjugate to the receiving zone of the test strip 1001. In the preferred embodiment of the invention, the combination of the components making up the receiving zone and the testing zone 1002 in the proper concentrations as defined herein, enables the test strip 1001 to determine whether a concentration of PdG above a pre-defined threshold exists within a sample of urine applied to the test strip 1001.

The present inventor has recognized that PdG levels in female urine range from 0.2-30 µg/ml throughout the typical menstrual cycle. Prior to ovulation, PdG levels in urine samples range from 0.2-5 µg/ml, and however PdG levels in urine samples increase to 5-30 µg/ml after ovulation. In an embodiment of the invention, the test strip 1001 is configured with a testing zone 1002 threshold of 5 µg/ml, such that when urine containing a concentration of PdG exceeding 5 µg/ml is applied to the test strip 1001, the test strip 1001 displays a positive result. In an embodiment, a positive result confirms that ovulation has taken place if the subject of the test is not pregnant. In contrast, in an embodiment, the test strip 1001 is configured such that when urine containing a concentration of PdG below 5 µg/ml is applied to the test strip 1001, the test strip 1001 displays a negative result.

In an embodiment of the invention, the test strip 1001 is configured to determine for the presence of PdG in a sample of urine applied to the test strip 1001 in an amount exceeding 5 µg/ml. In embodiments, the PdG-carrier protein conjugate is applied to the testing zone 1002 at a concentration level of a value selected from within the range of .5 µg/ml-2 µg/ml. In an embodiment, the PdG-carrier protein conjugate is applied to the testing zone 1002 at concentration level of .7 µg/ml.

In the invention, the testing zone 1002 is configured to comprise a progesterone metabolite, such as pregnanediol (PdG), conjugated to a Globulin carrier protein at a concentration of a value selected from the range of .5 µg/ml-2 µg/ml within the testing zone 1002. In an embodiment of the invention, the Globulin carrier protein consists of Bovine Gamma Globulin (BGG). The present inventor has recognized that the application of the PdG-carrier protein conjugate at the concentration levels described above, when applied to the test strip 1001 in conjunction with the application of the anti-PdG of a specified IgG isotype conjugated to a visual label of the specific concentration levels described herein, accomplishes the proper ratio of those specific binding partners to enable the test strip 1001 to detect for the presence of PdG in a sample of urine applied to the test strip 1001 at the pre-defined thresholds described above and further visually indicate that the sample of urine contains PdG above the pre-defined threshold or visually indicate that the sample of urine does not contain PdG above the pre-defined threshold.

Embodiments of the present invention are configured to measure additional analytes of progesterone in addition to PdG in the testing zone 1002. The present inventor has recognized that testing for and tracking multiple progesterone metabolites as opposed to merely testing for and tracking PdG provides a more accurate understanding of progesterone metabolism generally. The present inventor has further recognized that measuring additional analytes of progesterone (in addition to PdG) provides a more accurate representation of the level of serum progesterone on the test strip 1001 in an embodiment. Thus, in embodiments, the test strip 1001 is configured to measure progesterone metabolites other than and in addition to PdG in the testing zone 1001 and therefore more accurately provides an indication of ovulation. The present inventor has specifically noted that configuring a test strip 1001 by placing an anti-PdG antibody of the IgG isotype, optionally the IgG2b isotype, in the receiving zone facilitates the binding of other metabolites of progesterone aside from and in addition to PdG. Such other metabolites of progesterone include those selected from the list consisting of: sulfate conjugates of progesterone (PdS), 20(alpha)-hydroxyprogesterone, 20(beta)-hydroxyprogesterone, Sbeta-Pregnane-3(alpha), 17(alpha), 20(alpha)-triol, pregnanediol diacetate, allopregnanediol, epiallo-pregnanediol, and allo-pregnanediol disulphate. The present inventor has discovered that the antigen binding region associated with anti-PdG of the IgG isotype and its subtypes selected from IgG1, IgG2a, IgG2b and IgG2c, not only binds to PdG but to additional progesterone metabolites. Therefore, in an embodiment of the invention, the test strip 1001 is configured to detect additional metabolites of progesterone in addition to PdG to provide a more accurate representation of progesterone in serum and represents a significant departure from the prior art.

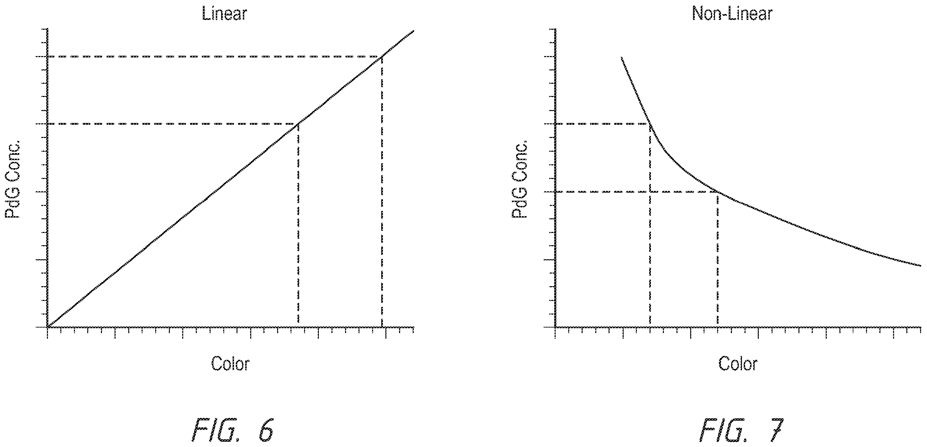

Due to its ability to detect multiple metabolites of progesterone, embodiments of the test strip 1001 differ from the prior art as embodiments of the test strip present results that are linear, as depicted in Fig. 6. The present inventor has determined that prior art attempts are less advantageous because the results associated with such prior art attempts, in contrast, are not linear. For instance, the present inventor has noted this problem with the prior art disclosed in United States Patent US 6924153 B1, issued to Quidel on August 2, 2005, which is that its non-linear nature would lead to results that were sometimes positive and sometimes negative for similar concentrations, decreasing the accuracy and robustness of the prior art. In association with the Quidel disclosure and others, the present inventor has noted that when antibodies that bind only to PdG, non-linear results were presented. However, in contrast, in association with embodiments of the present invention, upon utilization of antibodies that detected not only PdG but others, namely anti-PdG antibodies of the IgG isotype and subtypes, other progesterone metabolites were bound. Further the test strip 1001 is novel in its configuration as the anti-PdG of the IgG isotype presents the unique capability of binding to the label and presents results so that results can be viewed by the naked eye. Thus, in an embodiment, anti-PdG chosen for incorporation into the test strip has the unique characteristics in its antigen binding region making it uniquely adaptable to pregnanediol compounds other than PdG because it has the ability to bind other progesterone metabolites, and the additional characteristic of being able to bind to a label.

In an embodiment, the chosen anti-PdG antibodies of the IgG isotype and its subtypes contain conformational epitopes that allow for the antibody to bind not only PdG, but other metabolites of progesterone as well. The present inventor has discovered that when compared to other antibodies that solely bind to PdG, the chosen anti-PdG antibody of the IgG isotype capable of binding to other metabolites of progesterone produced linear and reproducible test results. Linear results, in association with embodiments of the invention, demonstrate high concentrations of PdG by depiction in the testing zone no visually detectible line; and medium concentrations of PdG by depiction in the testing zone 1002 of a faint line; and low or zero concentrations of PdG by depiction in the testing zone 1002 of a dark line. The linear relationship associated with embodiments of the invention is further depicted in Fig. 6. In other words, the level of PdG in urine directly correlates with the color intensity of the line displayed in the testing zone 1002 on the test strip 1001, up to a pre-defined threshold. The present inventor has recognized that, in embodiments of the invention, the anti-PdG antibodies of the IgG isotype and its subtypes contain conformational epitopes that recognize several different forms of pregnanediol compounds within urine along with PdG, and are thereby incorporated into the test strip 1001 in association with methods of manufacturing lateral flow assays as well understood by those skilled in the art.

A positive result is indicated by the visual display of one colored line on the test strip 1001, indicating the presence of PdG above the pre-defined threshold within the urine test sample applied to the test strip 1001. A negative result is indicated by the visual display of two colored lines on the test strip 1001, indicating the absence of PdG above the pre-defined threshold within the urine test sample applied to the test strip 1001. In various embodiments, the test strip 1001 is configured to display a positive indication or a negative indication based on a chosen pre-defined threshold configured by correspondingly altering the concentration level of at least one of the specific binding partners within the testing zone 1002, the receiving zone, or both the testing zone 1002 and the receiving zone.

The present inventor has recognized that such configuration is a novel improvement over other attempts, including the attempt disclosed in United States Patent US 6924153 B1, issued to Quidel on August 2, 2005, because the present embodiment enables the anti-PdG antibodies to bind immediately to the PdG within the applied sample of urine, rather than requiring the PdG to travel to the testing zone 1002 prior to binding as described, for instance, in the Quidel reference. Practically speaking, the present embodiment represents an improvement over the configuration disclosed in Quidel and elsewhere because it reduces the opportunity for other potential binding partners to compete with PdG to bind its antibody, thereby potentially increasing the efficacy of the test strip 1001 in the present embodiment as compared with previous attempts.

In various embodiments of the invention, the test strip 1001 comprises at least one specially configured receiving zone. In an embodiment, the receiving zone serves to receive a bodily fluid sample which may contain the metabolite of interest and to begin the flow of the sample along the test strip 1001. The receiving zone is prepared from a natural or synthetic porous or macroporous material which is capable of conducting lateral flow of the fluid sample. A porous or macroporous material suitable for purposes of this invention generally has a pore size greater than 12 µm. Examples of porous materials include, but are not limited to, glass, cotton, cellulose, nitrocellulose, polyester, rayon, nylon, polyethersulfone, and polyethylene. In an embodiment, the test strip 1001 is configured to comprise an anti-PdG antibody of the IgG isotype conjugated to a visual label at a concentration of a value selected from within the range of 1 µg/ml-10 µg/ml within at least one receiving zone. In an embodiment, the test strip 1001 is configured to comprise an anti-PdG antibody conjugated to a visual label at a concentration of 7 µg/ml within at least one receiving zone. In various embodiments, the visual label consists of colloidal gold. In various embodiments, the colloidal gold particles chosen to make up the anti-PdG antibody-collodial gold conjugate are of a size dimension of a value selected from the range of 20-100 nm. In various embodiments, the colloidal gold particles chosen to make up the anti-PdG antibody-collodial gold conjugate are of a concentration of 0.7-1.3 OD. The present inventor has recognized that the application of the anti-PdG conjugated to a visual label at the concentration levels described above, when applied to the receiving zone of the test strip 1001 in conjunction with the application of the PdG-carrier protein conjugate of the specific concentration levels described herein applied to the testing zone 1002 of the test strip 1001, accomplishes the proper ratio of those specific binding partners to enable the test strip 1001 to detect for the presence of PdG in a sample of urine applied to the test strip 1001, and further visually indicate that the sample of urine contains PdG above the pre-defined threshold or visually indicate that the sample of urine does not contain PdG above the pre-defined threshold.

Therefore, in various embodiments of the invention, the present inventor has recognized that the test strip 1001 configured as described effectively and reproducibly produces a negative test result if the PdG level in tested urine is below approximately 5 µg/ml; and the Test strip 1001 configured as described effectively and reproducibly produces a positive test result if the PdG level in tested urine is above approximately 5 mg/ml. The present inventor recognizes that due to variations in the ability of different populations to metabolize progesterone in urine, the 5 µg/ml threshold is not always the appropriate threshold. Therefore, various embodiments of the Test strip 1001 are configured to display a positive or negative result based on pre-defined thresholds of a PdG level of a value selected from values within the range of 3 µg/ml-20 µg/ml. The present inventor has recognized that embodiments of the invention are appropriately used in association with tracking progesterone levels during pregnancy, since as pregnancy progresses, pregnanediol levels also increase. Therefore, having a predetermined threshold of a higher value (e.g. 10 µg/ml - 20 µg/ml) allows the user to monitor pregnanediol levels through the duration of pregnancy. Such monitoring, with strips configured to measure pregnanediol with a pre-defined threshold from within the range of 10 µg/ml -20 µg/ml, allows the user to confirm that progesterone levels are increasing appropriately throughout pregnancy, and such configuration is incorporated as a teaching of an embodiment of the invention.

In the invention, PdG is conjugated to a specified Globulin as a carrier protein. The present inventor recognizes that non-globulin carrier proteins as utilized in prior art devices, including especially Bovine Serum Albumin (BSA), fail to enable adequate binding to the anti-PdG antibodies of the isotypes described herein, and thus fail to create an effective and reproducible testing mechanism. In embodiments of the invention, a specialized anti-PDG antibody isotype chosen specifically from the class of IgG isotypes more particularly described below, is conjugated to colloidal gold or similar visual label. The present inventor has recognized that only a highly specific subset of anti-PdG isotypes, said specific subset described further below, functions to provide an effective and reproducible urine testing mechanism for PdG. The invention as described herein, therefore, is presented as a solution to this and other persisting problems associated with previously known urine testing strips.

Embodiments of the invention comprise a method of creating a reproducible and effective test strip 1001, comprising the following steps. In the first conjugating step, PdG is conjugated to a specified Globulin as a carrier protein, and, separately, a mouse anti-PdG antibody chosen from the group of isotypes including IgG1, IgG1 Kappa, IgG2a, or IgG2c is conjugated to colloidal gold. In the configuring step, to create the Test strip 1001, the PdG-Globulin conjugate is impregnated or striped onto the nitrocellulose membrane in the testing zone 1002 of the test strip 1001. Colloidal gold conjugated anti-PdG antibody is applied or soaked into the receiving zone of the test strip 1001. When aurine sample is applied, the free PDG will bind to the anti-PDG antibody and travel to the testing zone 1002 of the test strip 1001. Any unbound anti-PDG antibody will bind to the testing zone 1002 area and produce a colored line. In this type of competitive assay format, the absence of color in the testing zone 1002 indicates a positive test result for the presence of a progesterone metabolite, and the presence of color in the testing zone 1002 indicates a negative result for the presence of a progesterone metabolite.

The present inventor has recognized that the use of Bovine Gamma Globulin (BGG), a Globulin, as a carrier protein represents an improvement over the use of other non-Globulin carrier proteins, such as BSA, in other known devices. Thus, embodiments of the present invention utilize BGG as the carrier protein. The present inventor has recognized that BGG has an ability to better bind to colloidal gold and thereby produce a more visually interpretable test result. On the other hand, the present inventor saw a need to create a test that preserves the benefit of BGG without the drawbacks noted in the Echogard et. al. study. Specifically, the inventor notes that the positive characteristics in the context of BGG's ability to not only bind better to colloidal gold, but to also to better present a PdG antigen to an antibody and to have a higher affinity to PdG. The present inventor notes that such advantages exist within the broader category of Globulins as carrier proteins, thus various embodiments of the invention utilize one of the Globulins as a carrier protein. These advantages are especially marked in comparison to the non-Globulin carrier protein BSA.

The present inventor has discovered that the drawback of utilizing a three zone immunoassay test strip 1001 as disclosed by Quidel for PdG quantification produces inconsistent results and does not meet the current needs of the market. In contrast, the preferred embodiment of the Test strip 1001 described herein, in contrast, is configured as a two zone immunoassay test strip 1001. The present inventor has determined that such a two zone immunoassay test strip 1001 produces more consistent results than strips configured as disclosed in Quidel and are therefore more accurate and reliable. Moreover, the increased simplicity and accuracy associated with the test strip 1001 as described herein as embodiments of the invention provides for increased compatibility and usability with test kits as described herein.

In the context of assays, the present inventor has recognized that immunoglobulins when used as carrier proteins can potentially bind additional proteins within a sample, creating potentially inconsistent or non-specific assay results for the analyte being evaluted. The present inventor has also recognized that with fewer assay components, the most robust and accurate the test strip 1001 will be. These advantages are viewed in stark contrast to the results observed in the 2013 Ecochard et. al. study, which involved a more complex device as described in the Quidel disclosure. Therefore, the present inventor has devised improvements from the discovered flaws associated with previously utilized prior art which are described in detail as embodiments of the invention disclosed herein.

The present inventor has discovered that to create a functional and reproducibly reliable urine test strip 1001 able to present clear results to the naked eye, or optionally able to present clear results to an external reader, a suitable anti-PdG antibody able to be conjugated to a visual label such as colloidal gold and/or latex beads, must be incorporated into an embodiment of the invention. The present inventor has discovered that the use of a mouse anti-PdG antibody of a peculiar and specific IgG isotype presents the desired characteristics and therefore is a teaching of the invention. In the preferred embodiment of the invention, the IgG2b isotype of mouse monoclonal anti-PdG antibody is utilized as the binding partner to a visual label. In alternative embodiments, the binding partner to a visual label comprises mouse anti-PdG antibody of the IgG1, IgG1 Kappa, IgG2a or IgG2c isotype. As referred to herein, the IgG2b, IgG1, IgG1 Kappa, IgG2a and IgG2c isotypes are subclasses of the IgG isotype, and are thereby incorporated as teachings of the invention.

The present inventor has recognized that such a specific combination uniquely allows for colloidal gold to be conjugated to the anti-PdG antibody of one of the specific isotypes mentioned above. Other combinations have been attempted, such as that described in Patent Application WO2016142610A1, which have failed to allow the colloidal gold to reproducibly function to produce the color needed to allow the test results to be viewable visually by the naked and untrained (layperson) eye. The present inventor has noted that the utilization of a Globulin conjugated to PdG allows for anti-PdG antibody of an immunologically active IgG isotype, selected from IgG1, IgG2a, IgG2b and IgG2c to bind in such a manner that colloidal gold is carried at a concentration sufficient for naked eye visualization, and is therefore a teaching of embodiments of the invention. The present inventor notes that Globulins evidence the preferable binding ratio of 8-32 PdG antigens per Globulin, which favor presentation of a visual result perceptible to the naked eye or to a reader, and is therefore a teaching of embodiments of the invention. The present inventor has recognized the benefit associated with embodiments of the invention described herein that a PdG test may be producible allowing the results to be visually interpreted with the naked eye.

Embodiments of the present invention comprise a Test strip 1001 configured to detect the presence of PdG in urine. In embodiments, the Test strip 1001 is optimized for visual detection by a layperson's, or non-expert's, naked eye while utilizing the Test strip 1001 in a non-laboratory context in association with intended purposes as described herein. The present inventor has recognized that in the invention, the Test strip 1001 is configured to comprise a combination of a mouse anti-PdG antibody of the IgG1, IgG2a, IgG2b, or the IgG2c isotype conjugated to a visual label, such as colloidal gold and/or latex beads, and PdG conjugated to an Globulin carrier protein, optionally BGG.

In embodiments of the invention, the carrier protein comprises one of the following human, non-human, or plant globulins: vicilin, legumin, casein, Alpha 1-antichymotypsin, seruam amylid A, Alpha 1-lipoprotein, Haptogolulin, Alphy 2-antiplasmin, Protein C, Angiotensinogen, cortisol binding protein, beta-2 microglobulin, plasminogen, angiostatins, sex-hormone-binding protein, transferrin, fibronectin, microglobuln, gamma globulin, thyroglobulin, 11S globulin family, 7S family of globulins. In various embodiments, the Globulin serving as the carrier protein derives from a plant or animal source, including an animal source such as human, mouse, rat, bovine, equine, goat, or rabbit. The present inventors has recognized that the resulting an embodiment of the invention configured as described herein comprises a visual test strip 1001 readable by the untrained eye in a context outside of a laboratory environment, as depicted in Figure 1.

The preferred embodiment of the current invention comprises a test strip 1001 comprising at least a testing area. Said testing area comprises a Globulin carrier protein conjugated with PdG, which is combined with an immunologically active mouse anti-PdG antibody of IgG2b isotype as a binding partner conjugated to colloidal gold. The conjugation of the Globulin carrier protein to the immunologically active mouse anti-PdG antibody takes place in association with methods known in the prior art, including methods known in United States Patent US 6924153 B1, issued to Quidel on August 2, 2005. The present inventor notes that such configuration preserves the ability associated with Globulins more generally to better present PdG antigen to an antibody, a higher affinity to PdG and better conjugation ratios of PdG to carrier - especially in comparison to the use of BSA as a carrier protein. Specifically, the present inventor has recognized that Globulin carriers more generally demonstrate the favorable conjugation ration of 8-32 antigens per one Globulin.

Such characteristics of Globulins specifically address the shortcomings identified with relation to the art described in WO 2016/142610, published on September 15, 2016, which discloses Bovine Serum Albumin (BSA) as the carrier protein utilized in association with a test for evaluating urine for PdG. The test disclosed in such does not have the ability to bind to colloidal gold, thereby resulting in a progesterone test that delivers results that are problematically imperceptible to the naked eye. In contrast, the present inventor has discovered that the urine PdG test disclosed herein utilizing a Globulin as the carrier protein also solves another related problem associated with prior art test attempts that utilize BSA, namely, that they are impractical for use with some readers that utilize light interpretation to determine the results for the test, because such tests incorporating BSA as a carrier protein fail to reliably produce enough color intensity to deliver perceptible test results.

The present inventors have recognized the benefit of a configuration of the preferred embodiment of the invention, embodied as a test strip 1001 configured to simultaneously analyze urine beyond mere analysis for the presence of pregnanediol, by further analyzing up to six (6) analytes and/or hormones in an alternative test strip 1001 configuration featuring a testing zone 1002 configured to evaluate urine for the presence of PdG beyond a specific threshold and optionally additional testing zones. In an embodiment, the test strip 1001 comprises a testing zone 1002 configured to evaluate urine for the presence of PdG beyond a specific threshold, a second testing zone 1003 and a third testing zone 1004. It is a teaching of an embodiment of the present invention for the test strip 1001 to optionally incorporate one or more additional testing zones beyond the testing zone configured to analyze urine for the presence of PdG, each additional testing zone specifically configured to evaluate urine for the presence of an item selected from the group consisting of LH, HCG, FSH, Testosterone and/or Estrogen or analytes thereof, such as E3G.

In an embodiment, the test strip 1001 is configured to simultaneously indicate, in a testing zone 1002, a positive or negative result for the presence of PdG above a pre-set threshold in a sample of urine applied to the test strip 1001, in addition to indicating, in a second testing zone 1003, a positive or negative result for the presence of at least one additional analyte and/or hormone, and, optionally, indicating in an additional third testing zone 1004, a positive or negative result for the presence of at least one additional analyte and/or hormone, all contained within a single test strip 1001. In an embodiment of the invention, the at least one additional analyte and/or hormone to be tested within the second testing zone 1003 and the at least one additional analyte and/or hormone to be tested within the third testing zone 1004 is selected from the following group: (1), Estradiol (E2) at concentrations 25-250 pg/ml in a competitive assay format; (2), Follicle Stimulating Hormone (FSH) at concentrations 3-20 mlU/ml in a sandwich assay format; (3), Luteinizing Hormone (LH) at concentrations 0-25 mlU/ml in a sandwich assay format; (4), Progesterone (P4) at concentrations of 0-40 ng/ml in a competitive or sandwich assay format; (5) human chorionic gonadotropin, (hCG) at concentrations of 0- 10,000 mlU/ml; and Testosterone, at concentrations of 0 to 50 µg, in a sandwich assay format. In an embodiment, the analyte and/or hormone that the test strip 1001 will simultaneously measure in at least a second testing zone 1003 and optionally a third testing zone are selected from the group consisting of E2, FSH, LH, P4, Testosterone and HcG. In an embodiment, the second testing zone 1003 is configured to detect for the presence of a hormone or analyte differing from the hormone or analyte detected by the third testing zone. In an embodiment, a digital reader 2001 is configured to evaluate the second testing zone 1003 and optionally the third testing zone 1004 in association with the methods further described herein. In an embodiment, the test strip 1001 further incorporates, in addition to a testing zone 1002, a second testing zone 1003, and a third testing zone 1004, a fourth testing zone configured to similarly detect for the presence of a hormone or analyte differing from the hormone or analyte detected by the other (first, second and third) testing zones within the test strip 1001. The methods of evaluation and further configurations optionally applied to the test strip 1001 associated with the testing of analytes and/or hormones beyond and in addition to pregnanediol are further described in United States Patent Application 15/974,229, filed on May 25, 2018.

In an embodiment of the invention, labels (such as colloidal gold) are varied, with a separate and distinct label configured to attach to a separate hormone. In such embodiment, the present inventor has recognized the advantage that the test strip 1001 is configured to provide a different color for each hormone analyte indicating either the presence or absence of each hormone analyte following application of urine to the test strip 1001. In an embodiment, the test strip 1001 is configured to comprise a conjugate pad (the conjugate pad also referred to as the "receiving zone" herein) comprising anti-PdG antibody-collodial gold conjugate, and at least one other conjugate. In an embodiment, the at least one other conjugate comprises anti-LH antibody-conjugated with a different label, optionally differently colored latex beads.

The present inventor has recognized that LH and HCG commonly exhibit cross-reactivity, specifically due to the fact that HCG can bind to LH antibodies. Therefore, having different colors corresponding to the presence of different hormones provides a benefit by allowing an observer to determine whether cross-reactivity has taken place. In other words, if an area designated to test for the presence of LH displayed the coloration of the label for HCG, one skilled in the art would understand such a read to indicate that cross-reactivity has been demonstrated and that the test therefore is invalid. Alternatively, the present inventor has noted that due to the similarities in structure between estrogen analytes and progesterone analytes (in at least one example, said progesterone analytes consisting of PdG), cross reactivity could take place between those two hormone metabolites specifically. Thus, it is beneficial to have different hormones or hormone metabolites labelled with different colors. Such labelling is accomplished in an embodiment by binding to colloidal gold and/or one or more differently colored latex beads, each testing zone within the strip featuring a differently colored label, and is therefore a teaching of an embodiment of the invention.

The present inventor has discovered that because PdG is a small hormone metabolite, in order to strongly bind to the surface of a membrane, PdG requires a strong carrier protein, which is a teaching of an embodiment of the invention. However, the present inventor has discovered that, for the preferred embodiment of the invention to function as intended, not only does the strong carrier protein need to bind the nitrocellulose membrane of the Test strip 1001, but the strong carrier protein also needs to bind the PdG and present it to the anti-PdG antibody, which is a teaching of an embodiment of the invention. Such teachings as disclosed herein, solve the challenges associated with suboptimal prior art teachings, which lacked the ideal combination of a strong carrier protein able to bind the PdG and present it to the anti-PdG antibody.

In accord with teachings of the invention, the test strip 1001 relies on the certain reagents being able to interact with other reagents to produce color in the testing zone 1002 of the membrane. Specifically, in the absence of PdG hormone in the urine sample, the following reagents must interact in order for the test results to be useful. First, in the preferred embodiment, colloidal gold must be conjugated to the immunologically active anti-PdG antibody of one of the specific IgG isotypes described elsewhere herein. In alternative embodiments, as a replacement for colloidial gold in other embodiments described herein, an alternative visual dye such as latex beads may be utilized to a similar effect. Further, in embodiments of the invention, the colloidal gold conjugated anti-PdG antibody must interact with the PdG-Globulin conjugate. Moreover, the PdG-Globulin must bind a nitrocellulose membrane. The present inventor has recognized that for these embodiments to function as intended, these interactions between and among the colloidal gold conjugated anti-PdG antibody and the PdG-Globulin conjugate, must be strong enough and stable enough to form and stay bound during urine sample application and lateral flow of urine across the reaction zone to solve the problems faced by the suboptimal prior art mechanisms described elsewhere herein. The disclosures in this paragraph constitute teachings of an embodiment of the invention.

In association with teachings of the invention, the test strip 1001 is configured to comprise a conjugate of Globulin with PdG. Such PdG-Globulin conjugate is combined with a mouse anti-PdG antibody of one the class of the IgG isotypes in an embodiment. The class of Ig isotypes includes IgG1, IgG2b, IgG2a or IgG2c isotype as contemplated in association with embodiments of the invention. The conjugation of a selection from within of a class of Globulins to PdG, and the combination of the PdG-conjugated Globulin with a mouse anti-PdG antibody of Ig isotype is accomplished in accord with general conjugation procedures as well-known by those skilled in the art.

It is a further teaching of the invention that in order for the preferred embodiment of the invention to function as intended, the specifically chosen anti-PdG antibody needs to be monoclonal, due to the nature of the PdG antigen presentation on the PdG-Globulin conjugate. In order for the embodiments of the invention to function as intended, the specifically chosen anti-PdG antibody must incorporate one of the following isotypes: IgG1, IgG2a, IgG2b, or IgG2c. The present inventor has discovered that isotypes other than IgG1, IgG2a, IgG2b, or IgG2c, including but not limited to IgM, IgS, and IgE anti-PdG antibody isotypes, remain unable to effectively bind the colloidal gold (or other visual label) and produce a strong enough color signal on the reaction zone due to their size and structure and are therefore excluded from the preferred embodiment of the invention. Since the colloidal gold must bind the Ig region of the anti-PdG antibody, the present inventor has discovered that the IgG1, IgG2a, IgG2b, and IgG2c isotypes of the anti-PdG antibody sufficiently bind colloidal gold and are therefore incorporated into embodiments of the invention. As a result, the IgG1, IgG2a, IgG2b and IgG2c isotypes of the anti-PdG antibody therefore produce the strongest color. In the preferred embodiment of the invention, the IgG2b isotype is included in the invention, as the present inventor has recognized that the IgG2b isotype performs slightly better when producing color. Therefore, the preferred embodiment of the invention incorporates the IgG2b isotype of the anti-PdG antibody. Alternative embodiments of the invention incorporate the IgG2a, IgG2c or IgG1 isotypes of the anti-PdG antibody.

The present inventor has recognized that the utilization of a Globulin within a specific combination uniquely allows for colloidal gold to be conjugated to the immunologically active anti-PdG antibody of one of the class of the IgG isotypes. In an embodiment, the combination enables the colloidal gold conjugated anti-PdG antibody to interact with the PdG-Globulin conjugate. In embodiments of the invention, therefore, the conjugate striped on the membrane in the testing area is PdG- Globulin and the anti-PdG antibody must be a monoclonal anti-PdG antibody of one of the following isotypes: IgG1, IgG2a, IgG2b, or IgG2c.

The present inventor recognizes that embodiments of the invention differ from other combinations that have been attempted in the prior art. Specifically, the other combinations have failed to allow the colloidal gold to function to produce the color needed to allow the test results to be viewable visually by the naked and untrained (layperson) eye. The present inventor has recognized that the novel utilization of a Globulin conjugated to PdG as described herein allows for anti-PdG antibody specifically of the Ig isotypes IgG1, IgG2a, IgG2b and IgG2c in accordance with the specific concentration levels described herein in embodiments of the invention, to bind in such a manner that colloidal gold is carried at a concentration sufficient for naked eye visualization. The present inventor has recognized the benefit associated with embodiments of the invention that a PdG test may be producible allowing the results to be visually interpreted with the naked eye and/or an external reader affordable to a typical consumer.

In embodiments of the invention, one carrier protein is conjugated to eight or more PdG molecules. In the preferred embodiment, the one carrier protein is conjugated to no more than thirty two PdG molecules. The present inventor has discovered that such a ratio allows for the colloidal gold conjugated anti-PdG antibody to bind with both enough affinity and avidity to produce a bright enough color in the test reaction zone for typical users to distinguish visually. The present inventor has discovered the specific property of Globulin enabling such combination. In embodiments of the invention, as Globulin exhibits the optimal number of active sites optimally spaced, the inclusion of Globulin results in a lesser amount of steric hindrance, and therefore embodiments of the invention are enabled to receive and bind PdG at sufficient ratios. Therefore, Globulin is essential for the preferred embodiment of the invention to function as intended. In the preferred embodiment of the invention, therefore, PdG is conjugated to a Globulin.

In association with teachings of the preferred embodiment of the present invention, a testing system to detect the presence of PdG is optimized for visual detection by a layperson's, or non-expert's, naked eye utilizing the embodiment in a context other than a professional laboratory environment. The present inventor has recognized that in embodiments of the invention, the combination of mouse anti-PdG antibody of an Ig isotype selected from isotypes IgG1, IgG2a, IgG2b and IgG2c conjugated to a visual label, such as colloidal gold and/or latex beads, and PdG conjugated Globulin as a carrier protein create sufficient binding partners to enable a test strip 1001 capable of visual inspection, or inspection by a reader as further described herein. Resultantly, the preferred embodiment of the invention comprises a visual test readable by the untrained eye in a context outside of a laboratory environment, as depicted in Figure 4.

In an embodiment of the invention, the method of creating the test strip 1001 comprises the following steps:
Selecting an immunologically active PdG antibody of a specific isotype selected from isotypes IgG1, IgG2a, IgG2b and IgG2c. In the preferred embodiment, the specific PdG antibody chosen is anti-PdG antibody of the IgG2b isotype. In alternative embodiments, the specific anti-PdG antibody chosen is one of the anti-PdG antibodies of either the IgG1, IgG2a, or IgG2c isotypes.

Conjugating the selected anti-PdG antibody to visual dye. Such step transforms the chosen immunologically active anti-PdG antibody into a visually labeled anti-PdG antibody. In the preferred embodiment, the visual dye consists of colloidal gold of a size value selected from within the range of 20-100 nm in diameter. In alternative embodiments, the visual dye comprises colloidal gold or colored latex beads.

Saturating the conjugate pad with the visually labeled anti-PdG antibody. In embodiments of the invention, the visual labelization takes place as a result of conjugating a anti-PdG antibody to a visual dye, such as colloidal gold.

Conjugating PdG to a Globulin carrier protein at a ratio of eight (8) or more units of PdG per 1 unit of Globulin. This ratio of units of PdG to units of Globulin constitutes a teaching of embodiments of the invention. In the preferred embodiment, the chosen Globulin carrier protein is BGG. In various embodiments, the Globulin is chosen from the list comprising: human, non-human, or plant globulins: vicilin, legumin, casein, Alpha 1-antichymotypsin, seruam amylid A, Alpha 1-lipoprotein, Haptogolulin, Alphy 2-antiplasmin, Protein C, Angiotensinogen, cortisol binding protein, beta-2 microglobulin, plasminogen, angiostatins, sex-homorne-binding protein, transferrin, fibronectin, microglobulin, gamma globulin, thyroglobulin, 11S globulin family, 7S family of globulins. In varying embodiments of the invention, globulins are chosen from plant or animal sources. Animal sources may include any from the list of: human, mouse, rat, bovine, equine, goat, or rabbit.

Impregnating PdG conjugated to the Globulin onto a test strip 1001 membrane at a chosen concentration level selected from the range of 0.5-2 mg/ml in a testing zone 1002, and
Impregnating anti-mouse antibodies into the control line 1005 area.

In the preferred embodiment, a test strip 1001 results from the above method.

The present inventor intends for the test strip 1001 to be used in a variety of scenarios and in association with a variety of methods. Such scenarios include utilization of the test strip 1001 as part of a method of quantifying and tracking PdG levels throughout the menstrual cycle. In accord with such method, the user will collect urine samples on different days of her menstrual cycle and use a test strip 1001 with each first morning urine, as depicted in Figure 3. Moreover, the present invention is useful to identify the fertile and non-fertile phase of a women's menstrual cycle.

Furthermore, the present inventor recognizes that if conception occurs, progesterone and PdG levels remain elevated throughout the duration of the pregnancy in a healthy pregnancy. Therefore, in association with a method of evaluation of whether the progesterone levels are such that a pregnancy may remain healthy, the test strip 1001 is intended to be utilized in association with a method of checking levels of progesterone during the pregnancy.

The test strip 1001 has other useful hormone monitoring uses. For example, the ovaries produce progesterone in large concentrations after ovulation has taken place. Therefore, lack of progesterone production over several weeks or months could indicate the start of menopause. Low amounts of progesterone could indicate an ovarian dysfunction such as failed ovulation attempt, luteinized unruptured follicle (LUF), or poor ovulation. Low or abnormal progesterone levels could also indicate a luteal phase defect. Additionally, in certain cancers such as breast, ovarian, and uterine, cancer cells are responsive or can secrete progesterone. In other methods of use, a female could utilize a test strip 1001 in association with pregnancy avoidance purposes. Specifically, a test strip 1001 showing a positive result indicates elevated PdG levels in urine, thereby indicating that the subject woman has ovulated and therefore entered her infertile phase.

In accordance with teachings of the presently contemplated test strip 1001, the present inventor has discovered a configuration including a unique combination of specific elements to allow for the detection of PdG in urine when applied to the test strip 1001 as further described herein. Such configuration allows a user to utilize the test strip 1001 determine whether PdG above a threshold amount is present in a urine sample applied to the test strip 1001. The present inventor has observed that the novel configuration presented herein demonstrates high specificity to PdG. The present inventor has recognized that the high specificity in association with PdG occurs due to the unique chemistry of the test strip 1001 especially in comparison to other attempts. Thus, the preferred embodiment of the test strip 1001s effectively and reproducibly provides an indication of whether a female has produced progesterone above a pre-defined threshold level.

In embodiments, the invention is characterized as an improvement to test kits known in the art. In such embodiments, the test strip 1001 as described herein is incorporated within a test kit in association a digital reader 2001.

A variety of test kits are contemplated for the application of the test strip 1001 described herein constituting an improvement thereof, which non-exhaustively may include the Clearblue^{®} Easy Fertility Monitor (CBEFM) that incorporates a means to interpret testing results and a digital display, which without a test strip 1001 configured to evaluate urine for pregnanediol (as further described herein) merely provides a method for monitoring the fertility status of an individual using two alternative hormones other than PdG: LH and E3G. Moreover, the test strip 1001 described herein is intended for use in association with a modified version of the Clearblue^{®} Digital Ovulation Test (CDOT), which without a test strip 1001 configured to evaluate urine for pregnanediol (as further described herein) merely employs a variable threshold for LH surge determination. Moreover, the test strip 1001 configured to evaluate urine for pregnanediol (as further described herein) is intended for use in association with a modified version of the First Response^{®} Advanced Digital Ovulation Test, which otherwise merely predicts the onset of ovulation by measuring LH and displaying a result. An embodiment of the invention comprises the CDOT or the First Response^{®} Advanced Digital Ovulation Test configured to comprise a test strip 1001 configured to evaluate urine for progesterone as described herein. In an embodiment, a digital reader 2001, optionally consisting of a stand-alone digital reader 2003, is further configured to read, store and present the results of such a test strip 1001. In an embodiment, a stand-alone digital reader 2003 is configured to wirelessly communicate with a smartphone digital reader 2002, optionally via Bluetooth, to store and present results from a test strip 1001 as collected by the stand-alone digital reader 2001 in association with an app operated by the smartphone digital reader 2002. The First Response^{®} Advanced Digital Ovulation Test, the CDOT, CBEFM, and similar devices are referred to as "stand-alone digital readers" 2003 as referred to herein. A stand-alone digital reader 2003 may comprise an input configured to read a test strip 1001 incorporated into a cartridge (also referred to herein as a "test stick" 2004). Examples of stand-alone digital readers 2003 are depicted in Figs. 3-5.

Further, the test strip 1001 configured to evaluate urine for pregnanediol as further described herein is intended for use in association with a variety of other digital readers, including a mobile device based system (such as a smartphone) that utilizes a camera 2005 to interpret the results of a lateral flow assay and which may utilize a variety of apps that track menstrual cycles. Embodiments of an improved test kit incorporating the test strip 1001 configured to evaluate urine for pregnanediol, and optionally other metabolites of progesterone, as further described herein are described below. The present inventor contemplates that additional configuration of test kits incorporating digital readers, aside from mere inclusion of the test strip 1001 configured to evaluate urine for the presence of pregnanediol, optionally and other metabolites of progesterone, is necessary to enable functionality and usefulness, in accordance with the methods and configurations as described herein.

An embodiment of the invention comprises a method of evaluating a subject's urine for the presence of PdG, such method including the steps of contacting a urine sample collected from a subject, typically a woman, with a test strip 1001. The method of evaluating urine for the presence of PdG includes a detecting the presence of PdG step, comprising determining that PdG of a concentration above a pre-defined threshold is present within the subject woman's urine by visually determining that one colored line is displayed on the test strip 1001, or determining that PdG of a concentration below a pre-defined threshold is present within the subject woman's urine by visually determining that two colored lines are displayed on the test strip 1001. During such method, during the detecting the presence of the PdG step, the visual display of one colored line on the test strip 1001 indicates that progesterone levels in the blood are elevated, and that ovulation has occurred in a non-pregnant woman. During such method, during the detecting the presence of the PdG step, the presence of two colored lines on the testing strip (including one colored line viewable within the testing area of the test strip 1001) indicates that progesterone levels in the blood are not elevated, and therefore indicates that ovulation has not occurred.

An embodiment of the invention comprises steps for determining whether a woman is in an infertile period by visually inspecting a test strip 1001 with the naked eye following the application of a sample of urine to the test strip 1001. In association with such method, the user may interpret the non-occurrence of ovulation, as indicated by a negative result on the test strip 1001, as a potentially fertile period.

An embodiment of the invention comprises steps for determining whether a pregnant woman has produced enough progesterone to sustain pregnancy. Said method comprises the steps of contacting a urine sample of women with a Test strip 1001 configured as further described herein, and detecting the presence of PdG by the absence of color in the testing area and the presence of color on a control line. During such method, during the detecting the presence of the PdG step, the absence of color in the testing area indicates that progesterone levels in the blood are elevated, and that a sufficient amount of progesterone has been produced to sustain a pregnancy. During such method, during the detecting the presence of the PdG step, the presence of color in the testing area indicates that progesterone levels in the blood are not elevated, and therefore indicates that a sufficient amount of progesterone may not have been produced to sustain a pregnancy. Following a result demonstrating that progesterone levels in the blood are not elevated, the subject of the test may pursue a mitigating step, whereby during the step of mitigating the subject of the test engages in the step of additional blood (serum) testing to determine with precision the specific level of progesterone in her blood, and/or a supplementing step to ingest, inject or otherwise add progesterone to her bloodstream in accordance with mechanisms known by those skilled in the art.

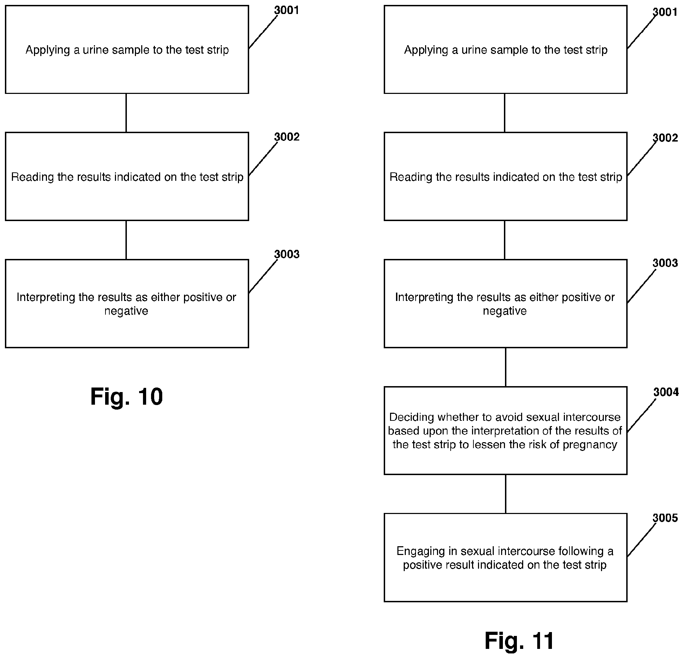

In an embodiment, the test strip 1001 is preferably utilized in accordance with the following steps: First, applying a urine sample 3001 to the test strip 1001. In association with the present method as depicted by Fig. 10, no additional treatment of the urine is required prior to application to the test strip 1001. Second, reading the results indicated 3002 on the test strip 1001 with the naked eye, following at least five minutes of waiting. Third, interpreting the results as either positive or negative 3003. In the preferred embodiment, a negative result is visually indicated by the presence of two colored lines on the test strip 1001. In an embodiment, a negative result is indicated by one colored line in the control line 1005 area, and one colored line in the testing zone area. In an embodiment, a positive result is visually indicated by the presence of only one colored line on the test strip 1001. In an embodiment, a positive result is displayed as the absence of a line in the testing zone 1002 area and the presence of a single line in the control line 1005 area.

In an embodiment of the invention, a positive result indicated on the test strip 1001, following the application of a sample of urine collected from a woman, determinable by the presence of only one visually perceptible colored line indicates that the woman whose urine was applied to the test strip 1001 was within her infertile period at the time the urine sample was applied to the test strip 1001. The present inventor intends for such information intended to be used in association with pregnancy avoidance purposes in a method of avoiding pregnancy while still optionally engaging in sexual intercourse, as depicted in Fig. 11. Therefore, an embodiment of the invention incorporating the steps described in the immediately preceding paragraph further comprises the step of interpreting the results as either positive or negative to contribute to a decision of whether to engage in sexual intercourse, said step also described as deciding whether to avoid sexual intercourse 3004. In said step, a user may decide to engage in sexual intercourse following a positive result 3005, indicating ovulation and that the woman was in an infertile period at the time of taking the test. Therefore, the woman could reasonably deduce that she could take the additional step of engaging in sexual intercourse without risk of pregnancy or a lessened risk of pregnancy immediately following a positive result indicated on the test strip 1001, and therefore may follow with an additional step of engaging in sexual intercourse following a positive result indicated on the test strip 1001. On the other hand, during the fourth step, a user may decide not to engage in sexual intercourse following interpreting the results as negative, which indicates that the user has not ovulated and therefore may still be in a fertile period.

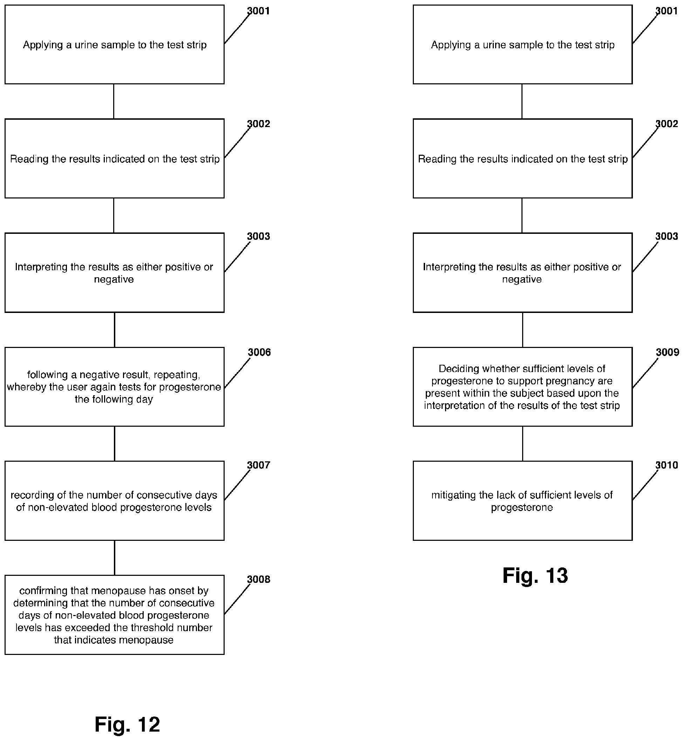

An embodiment of the invention comprises steps for determining whether menopause has occurred, as depicted in Fig. 12. Said method comprises the steps of contacting a urine sample 3001of women with a test strip 1001 configured as further described herein, and detecting the presence of PdG by reading the results indicated on the test strip 3002, alternatively presented as the presence or absence of color in the testing zone 1002 and the presence of color on a control line 1005. During such method, during the detecting the presence of the PdG step, also referred to as "interpreting the results as either positive or negative", 3003 the absence of color in the testing area (wherein a positive result is indicated by the presence of only one colored line on the testing strip) indicates that progesterone levels in the blood are elevated, and that menopause has not occurred. During such method, during the detecting the presence of the PdG step, the presence of color in the testing area (wherein a negative result is indicated by the presence of two colored lines on the test strip 1001) indicates that progesterone levels in the blood are not elevated. Following a result demonstrating that progesterone levels in the blood are not elevated (a negative result), the subject of the test engages in a repeating step 3006, whereby the user again tests for progesterone with the test strip 1001 the following day. For each repeating step, the user engages in a recording step 3007 to record the number of consecutive days of non-elevated blood progesterone levels. Finally, the user engages in a confirming step 3008, whereby the user confirms that menopause has onset by determining that the number of consecutive days of non-elevated blood progesterone levels has exceeded the threshold associated with menopause, such number of consecutive days of non-elevated blood progesterone levels indicative of menopause as known by those skilled in the art.

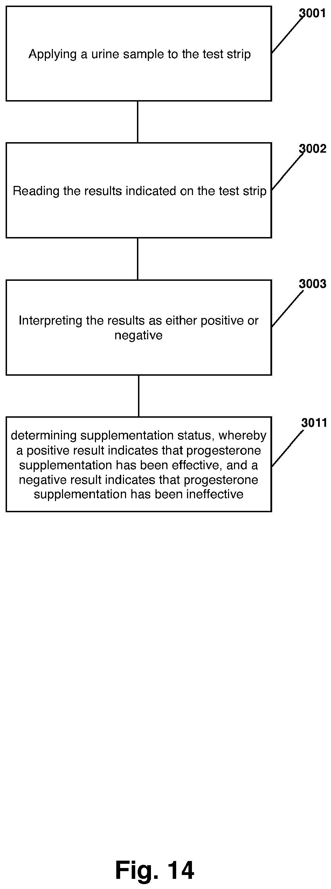

An embodiment of the invention comprises steps for determining whether progesterone supplementation is effective, as depicted in Fig. 14. The present inventor has noted that in cases where women are taking bioidentical progesterone supplements for the treatment of infertility, menopause, premenstrual syndrome (PMS) or any other health reason, the test strip 1001 can be used to determine if progesterone supplementation is effective. In an embodiment, a user engages in a supplementing progesterone step, wherein a user ingests, injects or otherwise adds progesterone supplements to the user's bloodstream. The user then engages in the steps of contacting a urine sample with a test strip 1001 configured as further described herein, and detecting the presence of PdG by the absence of color in the testing area and the presence of color on a control line. During such method, during the detecting the presence of the PdG step, also referred to as "interpreting the results as either positive or negative" 3003, the user then engages in the determining supplementation status step 3011, whereby a positive result indicates that progesterone supplementation has been effective, and a negative result indicates that progesterone supplementation has been ineffective.

An alternative embodiment of the invention comprises a system comprising a test strip 1001 and an external reader. In varying embodiments, such a system may be described as or otherwise form a portion of a "test kit."

In an embodiment, the external reader is configured to evaluate a test strip 1001. Following evaluation, the external reader is configured to display a positive result after evaluation of the test strip 1001 upon its determination that the testing zone of the test strip 1001 either does not contain any line of any luminance or color value, or otherwise contains a line perceptible only below a specified color value or luminance threshold as interpreted by an external reader.

In association with teachings of the invention, the test strip 1001 described herein may be utilized as part of a test kit incorporating a digital reader 2001. In an associated method, a first step of collecting urine takes place, wherein the user engages in a collecting step to collect at least one urine sample, or optionally a plurality of urine samples, each collected on different days of her menstrual cycle. For each sample collected, the user then engages in an applying step, where the user applies urine from the collected urine sample to a test strip 1001. The user then engages in an imaging step, wherein the user utilizes a digital reader 2001, optionally comprising a tablet computer or smartphone incorporating a built-in camera, to optically evaluate the at least one testing zone of the test strip 1001. The digital reader 2001 is configured, optionally by a software application, to perform a step of interpreting the results of either positive or negative by evaluating the presence of a line within the testing zone, or alternatively the intensity of the color or luminescence of the testing zone. Optionally, the digital reader 2001 is configured to compare the results evaluated from within the testing zone to the control line and/or the naked membrane, each of which optionally serves as a background measurement. During the interpretation of the results, the digital reader 2001 optionally generates a signal representative of the pregnanediol level indicated on the at least one test strip 1001.

In associated methods, a plurality of test strips 1001 are utilized during steps of quantifying and tracking pregnanediol levels throughout the menstrual cycle. In such methods, when such quantifying and tracking steps are performed in association with an external digital reader, each result may be compared to the other to track trends and map the user's menstrual cycle in association with steps and applications well known to those skilled in the art. In such way, the digital reader 2001 is configured to store a predetermined threshold associated with the level of pregnanediol, as previously collected pregnanediol levels remain in storage on the digital reader. In an embodiment, the associated methods include the methods disclosed in United States Patent Application 62/611,467 filed on December 28, 2017.

Optionally, the comparing step is performed by the digital reader, whereby the color intensities of the detection area are compared to other readings taken over time, and the software application is configured to determine significant increases or decreases in PdG concentrations. In an embodiment, the digital reader 2001 comprises a circuit configured to store at least one predetermined pregnanediol concentration threshold, generate a signal representative of the pregnanediol level indicated on at least one test strip 1001, compare the signal to a threshold and display a message indicating the result, as described further herein.

In an embodiment, a quantifying step follows, wherein the software application quantitates the concentration of PdG in the sample by comparing the intensity of the testing zone with the intensity of a known standard curve of PdG concentrations and line intensity. A displaying step then follows, where the software application presents a display of the test results on a screen incorporated into the digital reader, which may optionally include the word "ovulation" in association with a positive test and "no ovulation" in association with a negative test, on a screen perceptible to the user as the message indicating the result. As an alternative to the word "ovulation," the word "infertile," the phrase "safe to have sex," or a similar phrase may be displayed as the message indicating the result.

In most cases, diagnostic devices known in the art when evaluating hormones other than progesterone rely on the individual's hormone level to be either "high" or "low" relative to a fixed threshold value. In embodiments of the diagnostic devices described herein making use of the test strip 1001, an associated test kit comprises a test strip 1001 specially configured to be capable of evaluating urine for at least the presence of pregnanediol at or above a threshold level. When pregnanediol is determined as present in excess of the threshold level, it is a teaching of the invention to display a result related to "high," which optionally may include the word "ovulation" and/or "positive" as the message indicating the result. When progesterone is determined to be below this level, it is a teaching of the invention to display a result related to "low," which optionally may include the words "no ovulation" and/or "negative" display a message indicating the result. In an embodiment of the invention, the test strip 1001 is configured to evaluate urine for at least the presence of PdG at 5 µg/mL or greater. The configuration is so determined by the ratio of PdG-Globilun embedded into the testing zone to labeled anti-PdG antibody embedding into the test receiving zone. A "positive" or "high progesterone" reading will occur when color in the test receiving zone lighter than a certain threshold or no color exists. It has been found that many individual subjects do not conform to the average in terms of basal circulating hormone levels, cycle length and/or the duration of the cycle. Furthermore, variations can extend from one cycle to another in the same individual, making the use of a fixed threshold of PdG concentration as indicitave of a positive result for the entire population problematic. Thus, it is a teaching of the invention to modify the pregnanediol concentration threshold to accommodate populations for particular uses, or to store more than one pre-determined thresholds linked to specific populations on an associated digital reader, in accordance with the disclosures elsewhere herein.

In the CBEFM device described herein, which constitutes an example of a stand-alone digital reader 2003 in association with the disclosures herein, the user will monitor their urine sample over multiple menstrual cycles. The monitor stores the data, compares readings day to day, and identifies the days of maximum fertility. However, the CBEFM devices described above does not have the capability evaluate urine for the presence of PdG. These drawbacks are resolved in the inventive embodiments described herein, and it is a teaching of the present invention to apply the test strip 1001s to the CBEFM with modifications readily apparent to those skilled in the art, and utilize the CBEFM as a device for evaluation of the strips and the presentation of the results of the strips on a display associated with the CBEFM.

It is a teaching of the invention to incorporate a test strip 1001 into a test kit. A variety of examples for the implementation of the test kit comprising the specially configured single rest strip 1001 capable of evaluating urine for at least the presence of PdG are described herein. In one implementation, a test kit may include two units, a stand-alone digital reader 2003 and a package that contains multiple disposable test sticks. Because the test method can be effective in a single cycle, the kit may include only enough disposable test sticks 2004 for a single cycle of tests, for example, about 20. The reader may be activated mechanically, more preferably, activation is achieved by the change of the light reflectance of the background with or without the test stick 2004. The reader may also be designed to be activated by inserting a single-use disposable test stick 2004 and to measure the color development at the detection area of the test strip 1001 after a urine sample is applied. At the completion of the test, the test result may be converted to an electronic or digital output, viewable on a display 2010. The disposable test stick design 2004 may be based on lateral flow technology and contain the specially configured Test strip 1001 to evaluate urine for the presence of PdG.

Figure 3 depicts a stand-alone digital reader 2003 consisting of a testing zone 1002 of such a test kit without a disposable test stick 2004 installed in an embodiment. A stand-alone digital reader 2003 may be formed from plastic, metal, or other material. The stand-alone digital reader 2003 includes a test stick 2004 acceptor port 2020. The test strip 1001 acceptor port is designed to receive a test stick 2004 for analysis. The stand-alone digital reader 2003 also includes a display 2010. The display 2010 may render various icons or messages to a user such as test results, device status, or error messages. The display 2010 may be color or monochrome. In an example implementation, the display 2010 may be a liquid crystal display (LCD). In a variety of embodiments, the display 2010 may take a variety of configurations to effectively operate with a specially configured digital reader 2001. The stand-alone digital reader 2003 may further include a test stick alignment marker 2030. In the examples shown in Figs. 3-4, the test strip 1001 alignment marker 2030 is an arrow pointing to a corresponding arrow representing a test stick alignment marker 2031 on the test stick 2004. The test stick alignment marker aids with insertion of a test stick into the device 100. The device 100 may include a test stick ejector 140. The test stick ejector 140 may be a manual or electronic mechanism to eject a previously inserted test stick from the device 100.

Figure 4 depicts a stand-alone digital reader 2003 with a test stick 2004 inserted. In the example shown, the stand-alone digital reader 2003 is accepting a test stick 2004 housing the actual test strip 1001. It is desirable for the test stick 2004 to couple with the stand-alone digital reader 2003 so that the test stick 2004 will not fall out of the stand-alone digital reader 2003 and may form a water resistant seal to protect a portion of the stand-alone digital reader 2003 from fluid samples collected via the test stick 2004. The coupling should also minimize ambient light leakage into the stand-alone digital reader 2003 when testing is being performed on a test strip 1001. Fluid samples collected via the test stick 2004 are urine. An example test strip 1001 will be described below in reference to Figure 1. The test stick 2004 assembly includes a test stick housing. In an implementation, the test stick housing may be formed from plastic. The test stick assembly includes a test stick alignment marker 2031 corresponding with the test stick alignment marker on the stand-alone digital reader 2030. The test stick 2004 may also include a clicking sound feature to indicate proper alignment and insertion into stand-alone digital reader 2003.

An embodiment of the stand-alone digital reader 2003 may incorporate a printed circuit board. The printed circuit board includes one or more sensors. In an embodiment, the printed circuit board includes two optical sensors, configured to serve identical functions as a camera 2005 as described elsewhere herein. In this implementation, the sensors may be phototransistors. In other implementations, the sensors may be one or more photodiodes, electroactive sensors or radioactivity sensors. The sensors may be of the same or different types. The sensors are coupled with the processor chip.

The printed circuit board may include an emitter. In an implementation including photoelectric sensors, the emitter may be a light source such as a light emitting diode (LED). In an implementation including photoelectric sensors, the light source may be located equidistant between the photoelectric sensors. The light source may be coupled with the processor chip. The light source may illuminate according to a configurable pattern. In an implementation where the light source is coupled with the processor chip, the illumination pattern may be controlled by the processor chip. In an implementation where the light source is not coupled with the processor chip, the illumination pattern may be controlled by a separate timing circuit. In such embodiment, the light source allows the photoelectric sensors to read a test strip 1001 contained within a test stick 2004, and thereby perform identical functions to those performed by the camera 2005 as described elsewhere herein.

In an embodiment, as the emitter illuminates the test strip 1001, the sensor may detect a response from the illumination. For example, in an implementation where the emitter is a light source, the photoelectric sensor will detect the amount of light reflected by the test strip 1001. The test strip 1001 is configured to provide a positive result for at least the presence of PdG in urine by displaying the absence of a dark line in the testing zone 1002 that does not bind to colloidal gold in the preferred embodiment. Therefore, a positive reading (indicating the presence of PdG) will result in the absence of color or a reading below a certain color threshold reading off of the test strip 1001 by the photoelectric sensor. The photoelectric sensor therefore is configured to read a positive result upon determining that the reflection of light indicates a color level below a determined threshold. An example method of detection will be discussed in more detail below.

The emitter and sensor may be used to detect the insertion of a test stick 2004. When the stand-alone digital reader 2003 is not assembled with a test stick 2004, the emitter in the stand-alone digital reader 2003 can turn on periodically, for example, every two seconds. Detection of the presence of a test stick 2004 may be achieved by detecting a large difference in sensor response depending on whether the emitter is on or off due to the presence of the nearby reflective surface of the test stick 2004. For example, in an implementation including two photodiode sensors, four readings may be captured: (1) first sensor output with emitter on, (2) first sensor output with emitter off, (3) second sensor output with emitter on, and (4) second sensor output with emitter off. In this example, very low and approximately equal readings for all four indicate that the stand-alone digital reader 2003 is still in the packaging or sitting on the counter waiting for the next test to be performed. Readings indicating a high light intensity at the photodetector for tests 1 and 3 and a low light intensity at the photodetector for tests 2 and 4 indicate the presence of a test stick 2004. The stand-alone digital reader 2003 may use this information to alter operation mode (e.g., from low power stand-by mode in the packaging to higher power test mode when a strip is inserted).

In an embodiment, the sensor is positioned substantially over the testing zone 1002 of the test strip 1001 contained within a test stick 2004. A sensor may be positioned over a blank region downstream of the testing zone 1002 on the test strip 1001 in an embodiment. In this embodiment, no control/reference line is present. In an embodiment, reflectance measurements are made for these two regions for a time period after a fluid sample is applied to one end of the strip, optionally to determine presence of a sample.

The circuit includes a light emitter. The light emitter may be an LED. The light emitter is connected a processing/control circuit, optionally comprising a smartphone or tablet computer, that may be in the integrated circuit. The at least one photodetectors and are also each coupled to the processing/control circuit 806 to control initiation of the photodetector operation. In an embodiment, the at least one photodetector comprises a camera 2005, configured to obtain color data of at least one test strip 1001 configured to evaluate urine for the presence of pregnanediol, and a processor, configured to evaluate the color data obtained by the camera 2005. The processor is configured to evaluate a concentration value for each successive test strip 1001 based on the color data collected by the camera 2005 and to output a signal onto a display 2010.

It may be desirable to align the test strip 1001 when inserted into the device 100 such that the nitrocellulose region is substantially located under the sensor 430. A first sensor 430 may be located directly over the testing zone 1002. A second sensor may be located directly over a second region of the strip that may or may not contain a control line. Further details of one embodiment of a sensor 430, emitter 440, and Test strip 1001 alignment are discussed below. Measurements of the reflectivities provide a measure of PdG concentration. In an embodiment of the invention, the test strip 1001 is placed into a test stick comprising a rigid material, the test stick incorporating an opening such that the sensor can read the test strip 1001 through the test stick, and the test stick 2004 further configured to correspond to the dimensions of a port 2020 configured to accept the test stick 2004. Figure 3 depicts an embodiment of a test stick 2004 configured for placement into a corresponding port.

Turning now to PdG change detection methods which may be used by a digital reader 2001, optionally a stand-alone digital reader 2003, an implementation of an embodiment of the invention comprises the method for detecting a variation in a PdG level in a urine sample from an individual. At a step, a series of samples may be collected. Preferably, the series of samples is collected over a single biological cycle of the user, for example, over a single menstrual cycle. This allows a baseline and threshold to be developed in the same cycle. At a step, a baseline may be determined from a plurality of samples of the series collected. The baseline samples may be initial samples collected over an initial plurality of days of the cycle. For example, samples collected on the first three days after a user's menses may be used to determine a baseline. In an implementation, the samples may be collected on any one or more of the third through the tenth day from the onset of menses of a menstrual cycle of the individual.

At a step, a threshold may be associated with the PdG level for the individual based at least in part on the determined baseline. The threshold represents a personalized value for the specific individual monitoring the PdG level for a current biological status rather than relying on previously measured cycle information or a fixed threshold for all individuals. An example of one possible determination of a baseline and threshold will be described in further detail below. In an embodiment of the invention, the threshold value is the pre-determined threshold value, configured by the ratio of the specific binding partners of the test strip 1001.

At a step, a signal may be generated representative of the PdG level in one or more samples. For example, the signal may be based at least in part on an amount of light reflected by the testing zone 1002 on the test strip 1001, or lack of light reflected.

At a step, the signal may be compared to the threshold. The comparison may include detection of a difference between the values and/or statistical or probability analyses of the values. At a step, an output may be generated based at least in part on the comparing. Where the signal value drops below the threshold value, the amount of PdG may be higher in the current sample under test than the threshold value. In an embodiment of the invention, when the signal value drops below the threshold value, a positive result is indicated. This condition may correspond with an elevated level of PdG for the individual thereby confirming the occurrence of ovulation.

Embodiments of the digital reader 2001 comprise a camera 2005 and a processor. The camera may comprise a CMOS sensor or a CCD sensor able to sense photons from the electromagnetic spectrum, or colors displayed upon at least one test strip 1001. The processor performs an analysis of the coloration by processing the color information from the color space (RGB, sRGB, HUV, LAB), then transforming this information mathematically, for example, but not limited to, matrix multiplication, rotation, transposition normalization, etc., resulting in rotation, scaling and skewing of the values, to fulfil standard color spaces notation, or to non-conventional color spaces resulting from said mathematical transformations. The transformation may include a step for fixing the standard illuminant or adjusting the standard illuminant of said resulting color space. The transformation may result in a color space on which a coloration or set of colorations are represented as a larger fraction of the resulting color space. The analysis of the coloration of at least one test strip 1001 by the processor is used to assign hormonal concentration values to the colors detected by the camera. Then, a set of color values in the defined color space (for example R,G,B) is assigned a concentration value. In this way, concentration values are mapped to the analysed color space, thus leading to a high level of accuracy regarding different concentration values of PdG, and optionally other metabolites of progesterone, collected from a photographed test strip 1001. As the color values mapped to concentration values indicate the concentration of PdG within urine, the digital reader 2001 may then present a result of the test strip 1001 by displaying the progesterone concentration value corresponding to the color value presented to the user. The present inventor has discovered the unique attributes of the test strip 1001 as described herein that present a linear correlation between concentrations of PdG, and optionally other additional metabolites of progesterone, and color, such linear correlation necessary for the functioning of the digital reader 2001 to interpret the results of the test strip 1001.

According to the present embodiment, a pre-calibration step may be performed by the digital reader 2001 prior to reporting hormonal values. In this calibration step, the processor of the digital reader 2001 obtains color data from a set of test strip 1001 exposed to standard concentration values before a measurement cycle. At least two concentration values can be used as minimum, for example one that generates a dark line within the testing zone 1002 of the test strip 1001 indicating a negative result, and one that generates the maximum change in coloration within said testing zone 1002 with a faint line or no line, indicating a positive result. Preferably, a minimum of five concentration values is used. In order to decrease the concentration difference between the calibrated values and increase the accuracy of the measurement, the processor may be calibrated by using as many test strips 1001 as possible exposed to as many standard concentrations within the exposure range of the test strip 1001. In such way, the digital reader 2001 may update the mapping of the coloration value to the progesterone concentration value as indicated by the test strip 1001. In embodiments of the test strip 1001 containing a plurality of testing zones, the similar variations of the above process may be repeated to provide an accurate result for each testing zone.

The term "concentration value" also encompasses percentage values, or mathematical transformations of said values to any other numerical values, which mapped onto the color space may comprise further transformations, for example but not limited to logarithmic series, power series, prime series, or arbitrary number series for which one real concentration value corresponds to one mapped numerical value.

According to an embodiment, coloration and not intensity is measured by the digital reader. Measuring coloration of the test strips 1001 allows for the measurement to be performed under normal ambient conditions, for example, but not limited to, incandescent light, florescent light, day light, LED light, candle light, etc. The intensity of the light source under which the device performs a measurement can be expressed in terms of illuminance ranging from, but not limited to 4 1 lux to 4 1000 lux.

The digital reader 2001 then measures test strip 1001 values from strips exposed to urine containing hormones at different concentration values. The color information from the test strip 1001s is mapped to the color space as described above, and associated to a corresponding concentration from the calibration with standard concentrations. Then, the processor of the digital reader 2001 interprets the mapped color and associated concentrations to determine the PdG values indicated by the photographed test strip 1001s, optionally in association with an application used in association with the digital reader.

The digital reader 2001 then measures hormonal values over time and stores them within a circuit configured to store at least one predetermined pregnanediol concentration threshold, thereby allowing the digital reader 2001 to present positive or negative results. The values are collected from the test strip 1001 or from multiple test strips 1001 at time intervals, for example but not limited to, daily or weekly measurements. These measurements are collected for example, but not limited to, first morning urine.

The first upward PdG trend to be observed indicating a PdG concentration of 5 µg/ml after multiple measurements indicates that a woman has ovulated. The present inventor has discovered that a test strip 1001 in an embodiment of the invention configured to incorporate a testing zone able to detect metabolites of progesterone other than PdG provides a greater accuracy for depicting measurements of PdG, and therefore correspondingly, progesterone. The processor then may confirm that a first upward trend of the PdG has occurred by interpreting the successive test strip 1001 measurements, though subsequent negative readings may indicate the subsequent decline of progesterone following the initial production of progesterone following ovulation. Sustained levels of elevated progesterone readings, on the other hand, may indicate pregnancy. A single test indicating a PdG concentration at or above 5 µg/ml of PdG confirms ovulation.

Progesterone hormonal data obtained by the device may include test strip 1001 variability noise, or instrument noise, or ambient conditions variability noise. Noise reduction algorithms as well understood by those skilled in the art may be utilized to enhance the quality of data.

The digital reader 2001 may further comprise a display, configured to display the result of the test strip 1001. The user may be instructed on the display how to position the camera to obtain the color data of the test strip 1001. Thus, the user may obtain color data of the test strip 1001 with immediate feedback whether the color data suffices the requirements of the digital reader. Also, the results of the estimation may be displayed to the user immediately.

Progesterone and/or PdG data obtained by the device may include test strip 1001 variability noise, or instrument noise, or ambient conditions variability noise. Noise reduction algorithms, known to those skilled in the art, may be utilized to enhance the quality of data.

The test strip 1001 optionally additionally comprises a blank zone onto which no test samples are attached to, in addition to the testing zone and receiving zone, and wherein the processor may be configured to calibrate the device based on the obtained camera data of one or more of these zones. The test strip 1001s may comprise multiple testing zones, multiple blank zones, and multiple control line 1005s, wherein the processor may be configured to evaluate the camera data of these zones. The detection, control, and blank zones may vary in color or have the same color. The device may analyze these colors together or separately as described above.

In more detail, the control line 1005 is a zone onto which the applied test sample permeates, and which gives a clear indication whether the application of the test sample was successful. The blank zone is typically a white portion of the test strip 1001 which acquires a background coloration that may differ to its original color after exposure to the testing fluid. The testing zone is a zone onto which a coloration change is observed depending on the concentration value of pregnanediol in the test 'problem' sample. A concentration value associated with the sensitivity of the test strip 1001 corresponds in coloration to the control line 1005.

In an embodiment, the digital reader 2001 utilizes the coloration of the testing zone to be mapped to concentrations from the standard calibration described above, and indicate a pregnanedial concentration value, or an alternative message indicating the result, to a user. Furthermore, in an embodiment the color mapping to concentration can also be applied to the control line 1005 and blank zone and the device may utilize the control line 1005 and the blank zone to perform a local calibration on which the color information is utilized to evaluate the quality of the test strip 1001, the correct usage of the test strip 1001, or the illuminating conditions.

In an embodiment, the digital reader 2001 may be configured to calibrate the device based upon data stored from at least one previous measurement cycle of the device.

The present inventor recognizes that the progesterone levels of each woman and each cycle differ. In an embodiment, by evaluating the absolute progesterone levels of previous measurement cycles, the digital reader 2001 may estimate the base progesterone levels of the woman. Further, the digital reader 2001 may utilize the previous measurement cycles to estimate peaks of the progesterone levels. Depending on the estimated progesterone levels, the digital reader 2001 may determine a positive test indicating of progesterone and/or PdG levels above a threshold with higher accuracy. In such way, the digital reader 2001 confirms ovulation based on progesterone and/or PdG levels from each individual cycle and only uses previous cycle information to better calibrate for the detection of PdG based upon previous readings. In an embodiment, the digital reader 2001 also may be configured to read certain changes color levels that are not of a lacking intensity sufficient to confirm ovulation in the absolute, but such changes with a confirmatory blood test indicating progesterone production taken shortly before or after the urine progesterone test reading can allow the reader to calibrate to the color of the test strip 1001 at the time of the positive progesterone blood test to indicate a positive reading from a test strip 1001 in subsequent cycles.

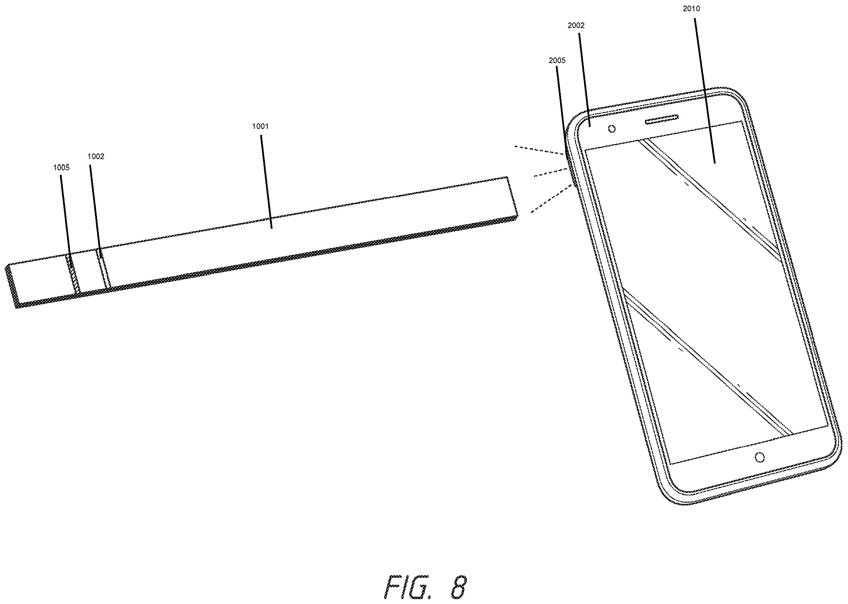

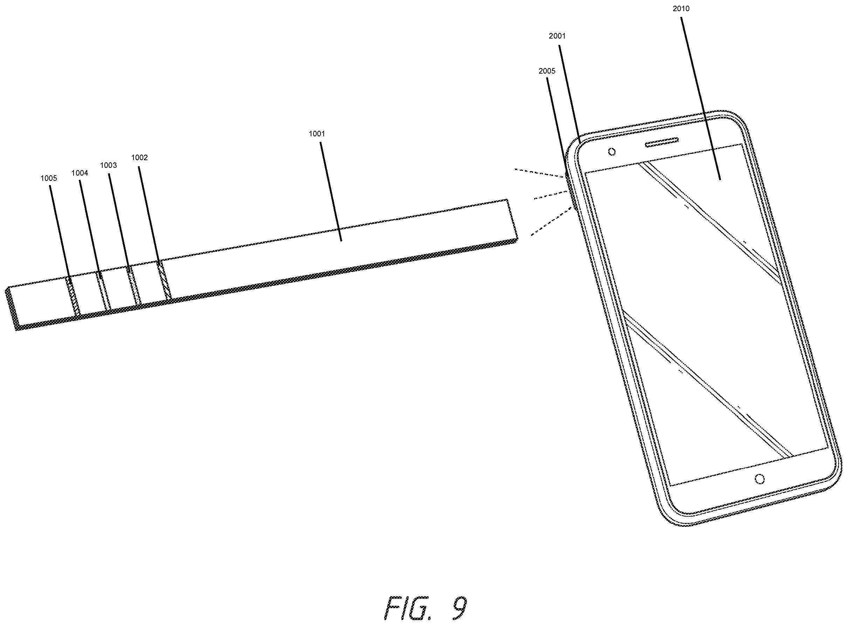

The digital reader 2001 may be configured as a smartphone, as depicted in Figs. 8 and 9. The display, the camera and the processor may be any known conventional display, camera 2005 and processor. The processor may be part of electric circuitry. The electric circuitry may be part of a controller. The controller may control the display and/or the camera. The display, the camera and the processor may be provided in the smartphone housing. The camera and the display may be provided in the smartphone housing in contact with the outside of the smartphone while the processor may be encapsulated in the smartphone housing.

Smartphones comprise cameras, processors, sensors and displays. Furthermore, smartphones are widely used today and carried by most people on a daily basis. By using a smartphone, the accuracy of high quality cameras for evaluation of the test strip 1001s may be utilized. Also, there is no need to provide an additional analyzation device. Smartphones also comprise a power supply such as a battery.

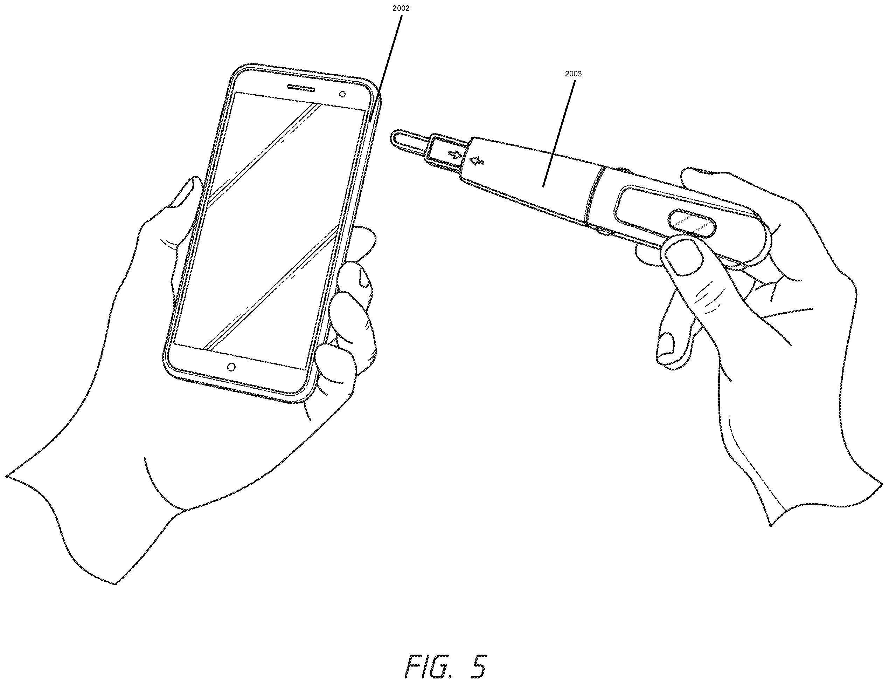

The digital reader 2001 may use multiple connectivity means, for example wifi, bluetooth, LTE, 3G, 4G, 5G to send and transmit data obtained or generated by the device elsewhere. In an embodiment depicted by Fig. 5, one digital reader may be configured to wirelessly transmit data collected to another digital reader consisting of a smartphone. Data may include results of the calculations or any other data associated with the usage of the embodiment of the invention, for example but not limited to, personal information, menstrual cycle information, location, time, and any other information provided by the device internal sensors or by user input. The test strip 1001 results collected in association with usage of the digital reader 2001 may be stored on the digital reader, or otherwise transmitted to any of the menstrual cycle tracking applications as known in the art. The present inventor further contemplates app-based PdG tracking, whereby levels of PdG are automatically or manually into an app in association with menstrual cycle prediction algorithms as known in the prior art.

The present inventor has recognized that women use menstrual cycle apps, so called "period-trackers" to help them gain insights into their menstrual cycles. Most of these apps are based off standard calendar methods, assuming women have a consistent luteal phase and ovulation date is often calculated as average menstrual cycle length - 14 days. However, this is only true for less than 20% of women, thus making the "rhythm method"-based ovulation dating highly inaccurate. Therefore, some of these apps incorporate additional fertility indicators such as cervical mucus observations, basal body temperature (BBT) recordings, ovulation predictor kits results (LH, FSH, E3G measurements), and cervical position. Since a urine PdG level of at least 5 µg/ml, utilized as the pre-determined threshold in an embodiment of the invention, is an indicator of ovulation, incorporating results of the test strip 1001 into period tracking apps known in the art will increase the accuracy of such at predicting ovulation. It is, therefore, a teaching of the invention for a digital reader 2001 to be utilized to transmit the results of the test strip 1001 into such period tracking apps in accordance with mechanisms known by those skilled in the art.

For confirmation of ovulation, the digital reader 2001 may be configured to output a signal onto a display 2010, if the processor determines that the test strip 1001 indicates the presence of PdG in urine above a pre-determined threshold. The signal may comprise a signal representative of the pregnanediol level indicated on the at least one test strip 1001. Alternatively, the signal may comprise a message. The message may state phrases such as "positive," "ovulation has occurred," "Ovulation has been confirmed", or "ovulation" to the user upon detecting a positive reading. On the contrary the signal indicated on the display 2010, for simplicity, may simply display "negative," "ovulation has NOT occurred," or "no ovulation" to the user upon detecting a negative reading.

Preferably, a wider color spectrum is obtained by the camera 2005 than by using conventional nearly monochromatic light sources and corresponding light receiving elements. Since the color indicating the amount of pregnanediol in the testing zone 1002 varies over the color spectrum, using a wider color spectrum enables that small changes in the amount of pregnanediol present in the testing zone 1002 can be determined more accurately than just a threshold intensity. Similar techniques are applicable to additional testing zones in embodiments of the invention. Thus, the concentration values in the sample to which a test strip 1001 is exposed can be determined more accurately, and therefore, a hormonal profile can be built using the digital reader 2001.

After calibration of the digital reader 2001, the color of the testing zone 1002 exposed to unknown concentration urine sample of the test strip 1001 is determined, and a pregnanediol value is assigned to the test strip 1001 depending upon the color in the testing zone 1002 and optionally translated into a signal, and the pregnanediol value is compared to the pregnanediol concentration threshold. The digital reader 2001 is then optionally configured to present a message onto a display 2010 indicating a result.

In an intended method of use, for monitoring the progesterone level, daily urine samples are applied to a series of test strip 1001, starting shortly after menstruation. Color data from each test strip 1001 in the series is obtained by the camera 2005 of the smartphone or other optical reading means of a digital reader 2001. In an embodiment, during the process of collecting color data of the test strip 1001, the camera 2005 of the smartphone is positioned a few centimeters and central over the test strip 1001 and a picture of the test strip 1001 is taken by the user or the color data or the test strip 1001 is processed by the smartphone 22 to compare the signal representative of the pregnanediol level indicated on the test strip 1001 to the predetermined pregnanediol concentration threshold. The smartphone may optionally be configured in association with methods understood by those skilled in the art to then present a message indicating the result on a display 2010.

In order to correct for different conditions such as different illumination etc., each color data of the test strip 1001 is subjected to a further calibration. For calibration, in an embodiment, the color of the blank zone of the test strip 1001, the blank zone optionally a portion of the receiving zone exclusive of the area of the testing zone 1002 and other testing zones, is compared with the color of the control line 1005 of the test strip 1001, which has maximum color change, induced by the urine sample. The first color (the dark line of the control line) is set to denote a minimum PdG value, while the second color (no line or a faint line) is set to denote a maximum PdG value.

The methods disclosed herein comprise one or more steps or actions for achieving the described method. The method steps and/or actions may be interchanged with one another without departing from the scope of the claims. The order and/or use of specific steps and/or actions may be modified without departing from the scope of the claims.

It is to be understood that the claims are not limited to the precise configuration and components illustrated above. Various modifications, changes and variations may be made in the arrangement, operation and details of the methods and apparatus described above without departing from the scope of the disclosure.

While the foregoing is directed to aspects of the present disclosure, other and further aspects of the disclosure may be devised without departing from the basic scope thereof.

## Claims

1. A test strip, configured to evaluate a sample of urine for the presence of pregnanediol (PdG) and display an indication of whether the sample of urine contains pregnanediol, wherein the test strip comprises:
a receiving zone comprising anti-pregnanediol antibodies of an IgG isotype conjugated to a visual label, wherein the IgG isotype is selected from IgG1, IgG2a, IgG2b, and IgG2c; and
a testing zone comprising pregnanediol conjugated to a Globulin carrier protein,
wherein the indication consisting of one line indicates a positive result, demonstrating that the sample of urine contains pregnanediol above a pre-defined threshold; and
wherein the indication consisting of two colored lines indicates a negative result, demonstrating that the sample of urine does not contain pregnanediol above a pre-defined threshold.

2. The test strip of Claim 1, further comprising a control line comprising anti-goat antibodies.

3. The test strip of Claim 1 or 2, wherein:
(a) the concentration of the anti-pregnanediol antibodies is 1-10 µg/ml, preferably wherein the concentration of the anti-pregnanediol antibodies is 5-9 µg/ml; and/or
(b) the anti-pregnanediol antibodies incorporate the ability to bind to other metabolites of progesterone aside from and in addition to PdG, preferably wherein the other metabolites of progesterone are selected from the group consisting of: Pregnanediol Sulfate (PdS), 20(alpha)-hydroxyprogesterone, 20(beta)-hydroxyprogesterone, 5beta-Pregnane-3(alpha), 17(alpha), 20(alpha)-triol pregnanediol diacetate, allopregnanediol, epiallo-pregnanediol, and allo-pregnanediol disulphate.

4. The test strip of any one of Claims 1 to 3, wherein the testing zone comprises:
(a) a nitrocellulose membrane configured to comprise two specific reagents bound in sequential order; and/or
(b) pregnanediol conjugated to a Globulin carrier protein at a concentration of a value selected from the range of 0.5 µg/ml-2 µg/ml.

5. The test strip of any one of the preceding claims, wherein the pre-defined threshold is selected from within the range of 3 µg/ml-10 µg/ml, preferably wherein the pre-defined threshold is set at 5 µg/ml.

6. The test strip of any one of the preceding claims, wherein the test strip is configured for interpretation by a digital reader.

7. A test strip, comprising:
a control line;
a receiving zone comprising anti-pregnanediol antibodies of an IgG isotype conjugated to a visual label, wherein the IgG isotype is selected from IgG1, IgG2a, IgG2b, and IgG2c;
a first testing zone configured to evaluate urine for the presence of pregnanediol, wherein the first testing zone comprises pregnanediol conjugated to a Globulin carrier protein; and
a second testing zone configured to evaluate urine for the presence of luteinizing hormone (LH).

8. The test strip of Claim 7, wherein:
(a) the first testing zone is further configured to evaluate urine for the presence of non-PdG metabolites of progesterone; and/or
(b) the test strip further comprises a third testing zone configured to evaluate urine for the presence of one of the hormones or metabolites selected from the group consisting of estrone-3-glucuronide (E3G), follicle stimulating hormone (FSH), Testosterone and human chorionic gonadotropin (hCG).

9. The test strip of any one of the preceding claims, wherein the visual label is colloidal gold.

10. The test strip of any one of the preceding claims, wherein:
(a) the Globulin carrier protein is Bovine Gamma Globulin (BGG); and/or
(b) the Globulin carrier protein is present at a binding ratio of 8-32 PdG antigens per Globulin.

11. A test kit for measuring the pregnanediol level in a urine sample, the test kit comprising:
a test strip of any one of claims 1 to 10;
a digital reader configured to capture results displayed test strip;
the digital reader further configured to interpret the results; and
the digital reader further comprising a display configured to present the interpreted results to the user.

12. The test kit of Claim 11, wherein:
(a) the digital reader is configured to:
capture results displayed on the test strip by sensing the colors
displayed on the test strip and analyzing the coloration;
interpret the results by comparing the coloration to a set of color
values in the defined pregnanediol concentration space; and present a result of the test strip by displaying the pregnanediol concentration value to the user; and/or
(b) the digital reader further configured to interpret the results of at least a second testing zone configured to evaluate for the presence of one of the hormones or metabolites selected from the group consisting of E3G, FSH, Testosterone, LH and hCG.

13. A test kit for detecting the presence or absence of pregnanediol above a threshold in a series of one or more urine samples collected from an individual, the test kit comprising:
at least one test strip of any one of claims 1 to 10, configured to evaluate urine for the presence of pregnanediol above or below a concentration threshold; and
a digital reader, the digital reader comprising:
a circuit configured to:
generate a signal representative of the pregnanediol level indicated on the at least one test strip;
compare the signal to the concentration threshold; and
display a message indicating the result.

14. The test kit of Claim 13, wherein:
(a) the digital reader comprises an optical sensor;
(b) the digital reader further comprises a light emitter and a camera; and/or
(c) the test strip is configured for housing within a test stick, and
a port configured to receive the test stick for analysis.

15. A method of utilizing and interpreting a test strip of any one of claims 1 to 10 configured to evaluate urine for the presence of pregnanediol, comprising:
applying a urine sample to the test strip,
reading the results indicated on the test strip,
interpreting the results as either positive or negative, wherein a positive result is indicated by the presence of one colored line on the test strip, and wherein a negative result is indicated by the presence of two colored lines on the test strip.

16. The method of Claim 15, further comprising:
(a) deciding whether to avoid sexual intercourse based upon the interpretation of the results of the test strip to lessen the risk of pregnancy, and
engaging in sexual intercourse following a positive result indicated on the test strip;
(b) deciding whether sufficient levels of progesterone to support pregnancy are present within the subject based upon the interpretation of the results of the test strip, and
following a negative result, mitigating the lack of sufficient levels of progesterone, by:
testing the subject's blood to determine with precision the specific level of serum progesterone, and
supplementing the subject with additional progesterone;
(c) following a negative result:
a repeating step, wherein the user tests again the following day,
a recording step, wherein the user records the number of consecutive days featuring a negative test result, and
a confirming step, wherein the user confirms that menopause has onset by determining that the number of consecutive days featuring a negative test result has exceeded the threshold number that indicates menopause; or
(d) a determining supplementation status step, whereby a positive result indicates that progesterone supplementation has been effective, and a negative result indicates that progesterone supplementation has been ineffective.

## Patentansprüche

1. Teststreifen, der konfiguriert ist, um eine Urinprobe auf die Anwesenheit von Pregnandiol (PdG) zu bewerten und eine Angabe darüber anzuzeigen, ob die Urinprobe Pregnandiol enthält, wobei der Teststreifen Folgendes umfasst:
eine Aufnahmezone, umfassend Anti-Pregnandiol-Antikörper eines IgG-Isotyps, konjugiert an eine sichtbare Markierung, wobei der IgG-Isotyp ausgewählt ist aus IgG1, IgG2a, IgG2b und IgG2c; und
eine Testzone, umfassend Pregnandiol, konjugiert an ein Globulin-Trägerprotein,
wobei die Angabe, die aus einer Linie besteht, ein positives Ergebnis angibt, was zeigt, dass die Urinprobe Pregnandiol über einem vordefinierten Schwellenwert enthält; und
wobei die Angabe, die aus zwei gefärbten Linien besteht, ein negatives Ergebnis angibt, was zeigt, dass die Urinprobe kein Pregnandiol über einem vordefinierten Schwellenwert enthält.

2. Teststreifen nach Anspruch 1, ferner umfassend eine Kontrolllinie, umfassend Anti-Ziege-Antikörper.

3. Teststreifen nach Anspruch 1 oder 2, wobei:
(a) die Konzentration der Anti-Pregnandiol-Antikörper 1-10 µg/ml beträgt, wobei bevorzugt die Konzentration der Anti-Pregnandiol-Antikörper 5-9 µg/ml beträgt; und/oder
(b) die Anti-Pregnandiol-Antikörper die Fähigkeit integrieren, an andere Metabolite von Progesteron abgesehen von und zusätzlich zu PdG zu binden, wobei bevorzugt die anderen Metaboliten von Progesteron ausgewählt sind aus der Gruppe bestehend aus: Pregnandiolsulfat (PdS), 20(Alpha)-Hydroxyprogesteron, 20(Beta)-Hydroxyprogesteron, 5Beta-Pregnan-3(alpha), 17(Alpha), 20(Alpha)-Triol-pregnandioldiacetat, Allopregnandiol, Epiallopregnandiol und Allopregnandioldisulphat.

4. Teststreifen nach einem der Ansprüche 1 bis 3, wobei die Testzone Folgendes umfasst:
(a) eine Nitrocellulosemembran, die konfiguriert ist, um zwei spezifische Reagenzien zu umfassen, die in sequenzieller Reihenfolge gebunden sind; und/oder
(b) Pregnandiol, konjugiert an ein Globulin-Trägerprotein in einer Konzentration eines Werts, ausgewählt aus dem Bereich von 0,5 µg/ml-2 µg/ml.

5. Teststreifen nach einem der vorhergehenden Ansprüche, wobei der vordefinierte Schwellenwert ausgewählt ist aus innerhalb des Bereichs von 3 µg/ml-10 µg/ml, wobei bevorzugt der vordefinierte Schwellenwert bei 5 µg/ml festgelegt ist.

6. Teststreifen nach einem der vorhergehenden Ansprüche, wobei der Teststreifen zur Interpretation durch ein digitales Lesegerät konfiguriert ist.

7. Teststreifen, umfassend:
eine Kontrolllinie;
eine Aufnahmezone, umfassend Anti-Pregnandiol-Antikörper eines IgG-Isotyps, konjugiert an eine sichtbare Markierung, wobei der IgG-Isotyp ausgewählt ist aus IgG1, IgG2a, IgG2b und IgG2c;
eine erste Testzone, die konfiguriert ist, um Urin auf die Anwesenheit von Pregnandiol zu bewerten, wobei die erste Testzone Pregnandiol umfasst, konjugiert an ein Globulin-Trägerprotein; und
eine zweite Testzone, die konfiguriert ist, um Urin auf die Anwesenheit von luteinisierendem Hormon (LH) zu bewerten.

8. Teststreifen nach Anspruch 7, wobei:
(a) die erste Testzone ferner konfiguriert ist, um Urin auf die Anwesenheit von nicht-PdG-Metaboliten von Progesteron zu bewerten; und/oder
(b) der Teststreifen ferner eine dritte Testzone umfasst, die konfiguriert ist, um Urin auf die Anwesenheit von einem der Hormone oder Metaboliten zu bewerten, ausgewählt aus der Gruppe bestehend aus Estron-3-glucuronid (E3G), follikelstimulierendem Hormon (FSH), Testosteron und humanem Choriongonadotropin (hCG).

9. Teststreifen nach einem der vorhergehenden Ansprüche, wobei die sichtbare Markierung kolloidales Gold ist.

10. Teststreifen nach einem der vorhergehenden Ansprüche, wobei:
(a) das Globulin-Trägerprotein Rinder-Gammaglobulin (BGG) ist; und/oder
(b) das Globulin-Trägerprotein in einem Bindungsverhältnis von 8-32 PdG-Antigene pro Globulin vorhanden ist.

11. Testkit zum Messen des Pregnandiol-Niveaus in einer Urinprobe, wobei das Testkit Folgendes umfasst:
einen Teststreifen nach einem der Ansprüche 1 bis 10;
ein digitales Lesegerät, das konfiguriert ist, um Ergebnisse zu erfassen, die auf dem Teststreifen angezeigt werden;
wobei das digitale Lesegerät ferner konfiguriert ist, um die Ergebnisse zu interpretieren; und
das digitale Lesegerät ferner eine Anzeige umfasst, die konfiguriert ist, um die interpretierten Ergebnisse dem Nutzer darzustellen.

12. Testkit nach Anspruch 11, wobei:
(a) das digitale Lesegerät konfiguriert ist für Folgendes:
Erfassen von Ergebnissen, die auf dem Teststreifen angezeigt werden, durch Erfassen der Farben, die auf dem Teststreifen angezeigt werden, und Analysieren der Färbung;
Interpretieren der Ergebnisse durch Vergleichen der Färbung mit einem Satz Farbwerten im definierten Pregnandiol-Konzentrationsbereich; und
Darstellen eines Ergebnisses des Teststreifens durch Anzeigen des Pregnandiol-Konzentrationswerts für den Nutzer; und/oder
(b) das digitale Lesegerät ferner konfiguriert ist, um die Ergebnisse von mindestens einer zweiten Testzone zu interpretieren, die konfiguriert ist, um auf die Anwesenheit von einem der Hormone oder Metaboliten zu bewerten, ausgewählt aus der Gruppe bestehend aus E3G, FSH, Testosteron, LH und hCG.

13. Testkit zum Erfassen der Anwesenheit oder Abwesenheit von Pregnandiol über einem Schwellenwert in einer Reihe von einer oder mehreren Urinproben, die aus einem Individuum gesammelt wurden, wobei das Testkit Folgendes umfasst:
mindestens einen Teststreifen nach einem der Ansprüche 1 bis 10, der konfiguriert ist, um Urin auf die Anwesenheit von Pregnandiol über oder unter einem Konzentrationsschwellenwert zu bewerten; und
ein digitales Lesegerät, wobei das digitale Lesegerät Folgendes umfasst:
eine Schaltung, die für Folgendes konfiguriert ist:
Generieren eines Signals, das das Pregnandiol-Niveau darstellt, das auf dem mindestens einen Teststreifen angegeben ist;
Vergleichen des Signals mit dem Konzentrationsschwellenwert; und
Anzeigen einer Nachricht, die das Ergebnis angibt.

14. Testkit nach Anspruch 13, wobei:
(a) das digitale Lesegerät einen optischen Sensor umfasst;
(b) das digitale Lesegerät ferner einen Lichtsender und eine Kamera umfasst; und/oder
(c) der Teststreifen konfiguriert ist zur Aufnahme in einem Teststab und
ein Anschluss, der konfiguriert ist, um den Teststab zur Analyse aufzunehmen.

15. Verfahren zum Verwenden und Interpretieren eines Teststreifens nach einem der Ansprüche 1 bis 10, der konfiguriert ist, um Urin auf die Anwesenheit von Pregnandiol zu bewerten, umfassend:
Auftragen einer Urinprobe auf den Teststreifen,
Ablesen der Ergebnisse, die auf dem Teststreifen angegeben werden,
Interpretieren der Ergebnisse als entweder positiv oder negativ, wobei ein positives Ergebnis durch die Anwesenheit von einer gefärbten Linie auf dem Teststreifen angegeben wird und wobei ein negatives Ergebnis durch die Anwesenheit von zwei gefärbten Linien auf dem Teststreifen angegeben wird.

16. Verfahren nach Anspruch 15, ferner umfassend:
(a) Entscheiden, ob Geschlechtsverkehr vermieden wird basierend auf der Interpretation der Ergebnisse des Teststreifens, um die Gefahr für eine Schwangerschaft zu verringern, und
(b) Entscheiden, ob ausreichende Mengen an Progesteron in dem Subjekt vorhanden sind, um eine Schwangerschaft zu unterstützen, basierend auf der Interpretation der Ergebnisse des Teststreifens, und
im Anschluss an ein negatives Ergebnis Mindern des fehlenden ausreichenden Progesteron-Niveaus durch:
Testen des Bluts des Subjekts, um genau das spezifische Niveau von Progesteron im Serum zu bestimmen, und
Supplementieren des Subjekts mit zusätzlichem Progesteron;
(c) im Anschluss an ein negatives Ergebnis:
einen Wiederholungsschritt, wobei der Nutzer am folgenden Tag erneut testet,
einen Aufzeichnungsschritt, wobei der Nutzer die Anzahl von aufeinanderfolgenden Tagen mit einem negativen Testergebnis aufzeichnet, und
einen Bestätigungsschritt, wobei der Nutzer bestätigt, dass Menopause begonnen hat durch Bestimmen, dass die Anzahl von aufeinanderfolgenden Tagen mit einem negativen Testergebnis die Schwellenwertanzahl überstiegen hat, die Menopause angibt; oder
(d) einen bestimmenden Supplementierungsstatusschritt, wobei ein positives Ergebnis angibt, dass Progesteronsupplementierung effektiv war, und ein negatives Ergebnis angibt, dass Progesteronsupplementierung ineffektiv war.

## Revendications

1. Bandelette de test, configurée pour déterminer la présence de prégnanediol (PdG) dans un échantillon d'urine et afficher une indication que l'échantillon d'urine contient du prégnanediol ou non, dans laquelle la bandelette de test comprend :
une zone de réception comprenant des anticorps anti-prégnanediol d'un isotype IgG conjugué avec un marqueur visuel, dans laquelle l'isotype IgG est sélectionné parmi IgG1, IgG2a, IgG2b, et IgG2c ; et
une zone de test comprenant du prégnanediol conjugué avec une protéine porteuse de globuline, dans laquelle l'indication constituée d'une ligne indique un résultat positif, démontrant que l'échantillon d'urine contient du prégnanediol en une quantité supérieure à un seuil prédéfini ; et
dans laquelle l'indication constituée de deux lignes colorées indique un résultat négatif, démontrant que l'échantillon d'urine ne contient pas de prégnanediol en une quantité supérieure à un seuil prédéfini.

2. Bandelette de test de la revendication 1, comprenant en outre une ligne de contrôle comprenant des anticorps anti-chèvre.

3. Bandelette de test de la revendication 1 ou 2, dans laquelle :
(a) la concentration en les anticorps anti-prégnanediol est de 1 à 10 µg/ml, de préférence dans laquelle la concentration en les anticorps anti-prégnanediol est de 5 à 9 µg/ml ; et/ou
(b) les anticorps anti-prégnanediol incorporent la capacité de se lier à d'autres métabolites de progestérone à part, et en plus de, PdG, de préférence dans laquelle les autres métabolites de progestérone sont sélectionnés parmi le groupe constitué de : sulfate de prégnanediol (PdS), 20(alpha)-hydroxyprogestérone, 20(bêta)-hydroxyprogestérone, 5bêta-prégnane-3(alpha), 17(alpha), 20(alpha)-triol, diacétate de prégnanediol, alloprégnanediol, épiallo-prégnanediol, et disulfate d'alloprégnanediol.

4. Bandelette de test selon l'une quelconque des revendications 1 à 3, dans laquelle la zone de test comprend :
(a) une membrane de nitrocellulose configurée pour comprendre deux réactifs spécifiques liés en ordre séquentiel ; et/ou
(b) du prégnanediol conjugué avec une protéine porteuse de globuline à une concentration d'une valeur sélectionnée dans la plage de 0,5 µg/ml à 2 µg/ml.

5. Bandelette de test de l'une quelconque des revendications précédentes, dans laquelle le seuil prédéfini est sélectionné dans la plage de 3 µg/ml à 10 µg/ml, de préférence dans laquelle le seuil prédéfini est établi à 5 µg/ml.

6. Bandelette de test de l'une quelconque des revendications précédentes, dans laquelle la bandelette de test est configurée pour interprétation par un lecteur numérique.

7. Bandelette de test, comprenant :
une ligne de contrôle ;
une zone de réception comprenant des anticorps anti-prégnanediol d'un isotype TgG conjugué avec un marqueur visuel, dans laquelle l'isotype IgG est sélectionné parmi IgG1, IgG2a, IgG2b, et IgG2c ;
une première zone de test configurée pour déterminer la présence de prégnanediol dans de l'urine, dans laquelle la première zone de test comprend du prégnanediol conjugué avec une protéine porteuse de globuline ; et
une deuxième zone de test configurée déterminer la présence d'hormone lutéinisante (LH) dans de l'urine.

8. Bandelette de test de la revendication 7, dans laquelle :
(a) la première zone de test est en outre configurée pour déterminer la présence de métabolites non-PdG de progestérone dans de l'urine ; et/ou
(b) la bandelette de test comprend en outre une troisième zone de test configurée pour déterminer la présence d'une des hormones ou d'un des métabolites sélectionnés parmi le groupe constitué de : estrone-3-glucuronide (E3G), hormone folliculostimulante (FSH), testostérone et gonadotrophine chorionique humaine (hCG) dans de l'urine.

9. Bandelette de test de l'une quelconque des revendications précédentes, dans laquelle le marqueur visuel est de l'or colloïdal.

10. Bandelette de test de l'une quelconque des revendications précédentes, dans laquelle :
(a) la protéine porteuse de globuline est gammaglobuline bovine (BGG) ; et/ou
(b) la protéine porteuse de globuline est présente à un rapport de liaison de 8 à 32 antigènes PdG par globuline.

11. Kit de test pour mesurer le niveau de prégnanediol dans un échantillon d'urine, le kit de test comprenant :
une bandelette de test de l'une quelconque des revendications 1 à 10 ;
un lecteur numérique configuré pour capturer des résultats affichés sur la bandelette de test ;
le lecteur numérique étant en outre configuré pour interpréter les résultats ; et
le lecteur numérique comprenant en outre un écran d'affichage configuré pour présenter les résultats interprétés à l'utilisateur.

12. Kit de test de la revendication 11, dans lequel :
(a) le lecteur numérique est configuré pour :
capturer des résultats affichés sur la bandelette de test en détectant les couleurs affichées sur la bandelette de test et en analysant la coloration ;
interpréter les résultats en comparant la coloration à un ensemble de valeurs de couleur dans l'espace défini de concentration en prégnanediol ; et
présenter un résultat de la bandelette de test en affichant la valeur de concentration en prégnanediol à l'utilisateur ; et/ou
(b) le lecteur numérique en outre configuré pour interpréter les résultats d'au moins une deuxième zone de test configurée pour déterminer la présence d'une des hormones ou d'un métabolites sélectionnés parmi le groupe constituée de : E3G, FSH, testostérone, LH et hCG.

13. Kit de test pour détecter la présence ou l'absence de prégnanediol en une quantité supérieure à un seuil, dans une série d'un ou de plusieurs échantillons d'urine prélevés chez un individu, le kit de test comprenant :
au moins une bandelette de test de l'une quelconque des revendications 1 à 10, configurée pour déterminer la présence de prégnanediol, dans de l'urine, en une quantité supérieure ou inférieure à un seuil de concentration ; et
un lecteur numérique, le lecteur numérique comprenant :
un circuit configuré pour :
générer un signal représentatif du niveau de prégnanediol indiqué sur l'au moins une bandelette de test ;
comparer le signal au seuil de concentration ; et
afficher un message indiquant le résultat.

14. Kit de test de la revendication 13, dans lequel :
(a) le lecteur numérique comprend un capteur optique ;
(b) le lecteur numérique comprend en outre un émetteur de lumière et une caméra ; et/ou
(c) la bandelette de test est configurée pour le logement à l'intérieur d'une baguette de test, et
un orifice est configuré pour recevoir la baguette de test pour l'analyse.

15. Procédé d'utilisation et d'interprétation d'une bandelette de test de l'une quelconque des revendications 1 à 10 configurée pour déterminer la présence de prégnanediol dans de l'urine, comprenant :
l'application d'un échantillon d'urine sur la bandelette de test,
la lecture des résultats indiqués sur la bandelette de test,
l'interprétation des résultats comme étant soit positifs soit négatifs, dans lequel un résultat positif est indiqué par la présence d'une ligne colorée sur la bandelette de test, et dans lequel un résultat négatif est indiqué par la présence de deux lignes colorées sur la bandelette de test.

16. Procédé de la revendication 15, comprenant en outre :
(a) la décision qu'il faut ou non éviter des rapports sexuels, sur la base de l'interprétation des résultats de la bandelette de test pour réduire le risque de grossesse, et
l'engagement dans des rapports sexuels suivant un résultat positif indiqué sur la bandelette de test ;
(b) la décision que des niveaux suffisants de progestérone pour soutenir la grossesse sont présents ou non chez le sujet, sur la base de l'interprétation des résultats de la bandelette de test, et
suivant un résultat négatif, la mitigation du manque de niveaux suffisants de progestérone en :
effectuant un test du sang du sujet pour déterminer avec précision le niveau spécifique de progestérone sérique, et
fournissant, au sujet, une supplémentation en progestérone ;
(c) suivant un résultat négatif :
une étape de répétition, dans laquelle l'utilisateur effectue à nouveau un test le jours suivant,
une étape d'enregistrement, dans laquelle l'utilisateur enregistre le nombre de jours consécutifs présentant un résultat de test négatif, et
une étape de confirmation, dans laquelle l'utilisateur confirme que la ménopause a commencé en déterminant que le nombre de jours consécutifs présentant un résultat de test négatif a dépassé le nombre seuil qui indique la ménopause ; ou
(d) une étape de détermination d'état de supplémentation, moyennant quoi un résultat positif indique que la supplémentation en progestérone a été efficace, et un résultat négatif indique que la supplémentation en progestérone a été inefficace.
